# EUROPEAN PATENT APPLICATION

(11) **EP 4 190 916 A1**
(43) Date of publication of application: **07.06.2023**
(21) Application number: 21851037.8
(22) Date of filing: 02.07.2021
(51) Int. Cl.: C12Q 1/6883, G01N 33/68, A61K 38/00, A61P 21/00

(54) **COMPOSITION FOR DIAGNOSING MUSCULOSKELETAL DISEASES, COMPOSITION FOR PREVENTING OR TREATING MUSCULOSKELETAL DISEASES, AND USE THEREOF**

(30) Priority: 31.07.2020 KR 20200095891
(71) Applicant: Seoul National University Hospital, Seoul 03080 (KR); Seoul National University R & DB Foundation, Seoul 08826 (KR); Cheongju University Industry & Academy Cooperation Foundation, Cheongju-si, Chungcheongbuk-do 28503 (KR)
(72) Inventor: JO, Hyun Chul, Seoul 05502 (KR); HWANG, Young-il, Seoul 01429 (KR); KIM, Jin-Hee, Cheongju-si Chungcheongbuk-do 28502 (KR); KIM, Jin-Hong, Seoul 08797 (KR); LEE, Jae-Hyung, Seoul 06010 (KR); LIM, Hyun-Ju, Jeonju-si Jeollabuk-do 55111 (KR); LEE, Ah-Young, Anyang-si Gyeonggi-do 14043 (KR); LEE, Seung Yeon, Seoul 08354 (KR); YEA, Ji-Hye, Hwaseong-si Gyeonggi-do 18530 (KR); KIM, Yeasol, Seoul 06917 (KR)
(74) Representative: V.O.
(86) International application number: PCT/KR2021/008404
(87) International publication number: WO 2022/025456

(57) **Abstract**

The present disclosure relates to a composition for diagnosing a musculoskeletal disease and a composition for preventing or treating a musculoskeletal disease. Zkscan8, which is presented in the present disclosure, can be effectively used as an excellent biomarker for obtaining accurate information about the occurrence and progression stages of a musculoskeletal disease, specifically a tendon disease or a ligament disease. In addition, the composition of the present disclosure can be effectively applied as a composition for preventing or treating a musculoskeletal disease through Zkscan8 overexpression.

## Description

### [Technical Field]

The present disclosure relates to a Zkscan8 gene related with tendons, ligaments, muscles, cartilages, nerves and bones. It relates to a method for diagnosing and treating corresponding musculoskeletal diseases by measuring the expression level of Zkscan8 and regulating its expression based on the new finding that it is decreased in subjects with musculoskeletal diseases and the recovery of musculoskeletal tissues and cells is promoted by its overexpression.

### [Background Art]

According to the US statistics, more than 20 million cases of musculoskeletal disorders occur every year, the most common causes being sprains, fractures, contusions, etc. The total cost for treating the musculoskeletal disorders or diseases is about 150 billion dollars per year.

When the musculoskeletal system is damaged by inflammations caused by aging (degenerative change) or trauma or partial or complete ruptures, no special therapeutic method is available yet except for surgical treatments for suturing, medications for alleviating symptoms such as steroid injections, and physical therapies.

In particular, the medications merely alleviate topical inflammations by attenuating pro-inflammatory factors. Nonsteroidal anti-inflammatory drugs (NSAIDs) such as indomethacin, ketoprofen, ibuprofen, acetylsalicylic acid and flurbiprofen and steroidal drugs commonly used. The nonsteroidal anti-inflammatory drugs are disadvantageous in that long-term medication can cause gastrointestinal side effects, and the steroidal drugs are also known to cause side effects such as osteoporosis, hypertension, neurological complications, etc. during long-term use.

In order to solve these problems, cell therapy agents that can treat musculoskeletal diseases of tendons, muscles, cartilages, nerves, bones, etc. through more fundamental tissue regeneration are drawing a lot of attentions.

Although a therapeutic agent for treating muscular diseases using an angiotensin II type1 receptor blocker and adipose-derived stem cells as active ingredients is known, its efficacy cannot be ensured because the differentiation of the stem cells is not determined by the angiotensin receptor blocker.

In addition, although a therapeutic agent using bone marrow-derived mesenchymal stem cells and adipose-derived mesenchymal stem cells, which improved the structuration of collagen tissues and collagen fibers and improved the tensile strength of tendons in a rat model of rotator cuff injury, as active ingredients is known, abnormal tissue change that increases the activity of alkaline phosphatase and induces heterotopic bone formation was observed in the regenerated tendons. There is a problem that the abnormal tissue change causes side effects such as increased re-rupture of tendons and delayed recovery.

Induction of normal tissue regeneration is required to solve these problems. For this, it should be possible to control mesenchymal stem cells as desired through physical stimulation, coculture with other cells, scaffolding, introduction of biologically active substances or differentiation-inducing genes, etc.

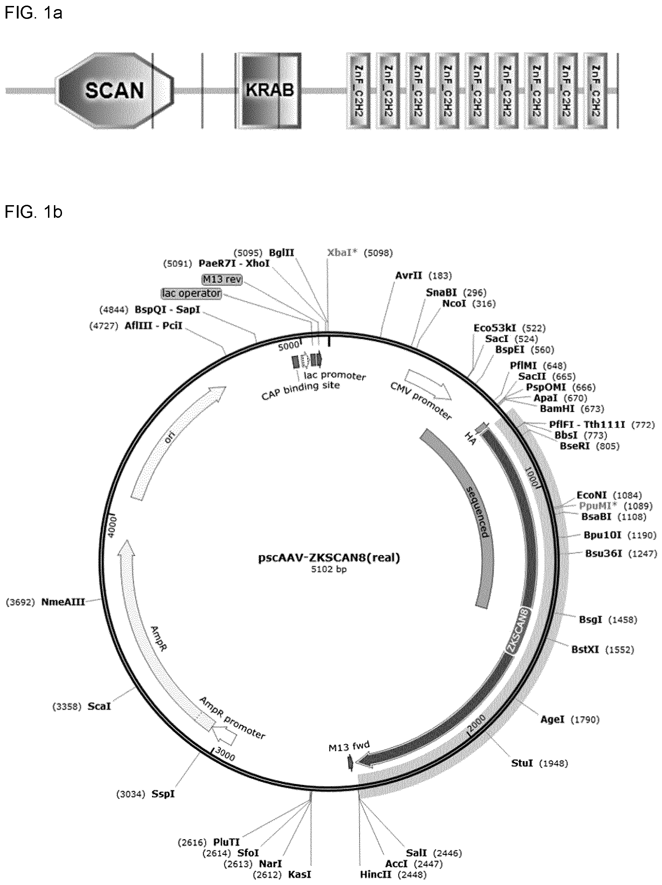

Zkscan8 (zinc finger protein with KRAB and SCAN domains 8) is a class of C2H2-ZNF (zinc finger) protein transcription factor. It is a transcription factor having a KRAB (Kruppel-associated box) domain and SRE-ZBP and SCAN (CTfin51, AW-1 and Number 18 cDNA) domains towards the N-terminal (FIG. 1a).

ZFPs (zinc finger proteins) are reported to have various biological activities. In particular, it is known to be expressed in various early progenitor cells including embryonic stem cells (ESCs). It is known that ZNF 589, 268 and 300 affect the differentiation of hematopoietic stem cells. However, the biological characteristics of Zkscan8 (ZNF 192, LD5-1, Zscan 40) have not been reported yet. Particularly, the function of Zkscan8 in the musculoskeletal system has not been researched yet.

The inventors of the present disclosure have identified the potential of Zkscan8 in diagnosis and treatment of the diseases of the musculoskeletal system, and have completed the present disclosure by identifying its effect on the diagnosis, prevention and treatment of musculoskeletal diseases.

Throughout the present specification, a number of papers and patent documents are referred to and their citations are indicated. The disclosed contents of the cited papers and patent documents are included herein by reference in their entirety, so that the level of the related art and the contents of the present disclosure can be described more clearly.

### [References of Related Art]

### [Patent Documents]

(Patent document 1) Korean Patent Registration No. 1,219,624.

### [Disclosure]

### [Technical Problem]

The inventors of the present disclosure have made consistent efforts to find out a biomarker related with musculoskeletal diseases, specifically tendon, muscle, cartilage, nerve and bone diseases. As a result, they have identified that Zkscan8 (zinc finger protein with KRAB and SCAN domains 8) is decreased in tendon, muscle, cartilage, nerve and bone diseases and have completed the present disclosure by finding out that musculoskeletal diseases can be prevented, ameliorated or treated by overexpressing Zkscan8.

The present disclosure is directed to providing a composition for diagnosing a musculoskeletal disease.

The present disclosure is also directed to providing a method for providing information for diagnosing a musculoskeletal disease.

The present disclosure is also directed to providing a composition for preventing or treating a musculoskeletal disease.

The present disclosure is also directed to providing a method for screening a composition for preventing or treating a musculoskeletal disease.

The present disclosure is also directed to providing a Zkscan8 (zinc finger protein with KRAB and SCAN domains 8) gene for preparation of a drug or a veterinary drug for treating a musculoskeletal disease, a vector including the gene and a novel use of a composition containing a cell including the vector or a culture thereof as an active ingredient.

Other purposes and advantages of the present disclosure become more apparent by the following detailed description, claims and drawings.

### [Technical Solution]

In an aspect of the present disclosure, the present disclosure provides a composition for diagnosing a musculoskeletal disease, which contains a Zkscan8 (zinc finger protein with KRAB and SCAN domains 8) gene or an agent for measuring the expression level of a protein encoded by the gene as an active ingredient.

The inventors of the present disclosure have made consistent efforts to find out a biomarker which provides comprehensive and highly reliable information for diagnosis of musculoskeletal diseases, specifically tendon, muscle, cartilage, nerve and bone diseases and can be measured easily with body fluid analysis only without a tissue sample. As a result, they have found out that Zkscan8 (zinc finger protein with KRAB and SCAN domains 8) is expressed specifically in the musculoskeletal system and its expression is decreased when a musculoskeletal disorder or disease occurs. As a result, they have found out that it can function as a superior biomarker for diagnosis of a musculoskeletal disease by providing accurate information about the occurrence and progression stages of a musculoskeletal disease.

In the present specification, the term "diagnosis" encompasses determining the susceptibility of a subject to a specific disease, determining whether a subject currently has a specific disease and determining the prognosis of a subject having a specific disease. Since the Zkscan8 (zinc finger protein with KRAB and SCAN domains 8) gene of the present disclosure or the expression level of a protein encoded by the gene is a genetic marker for not only the current state but also the risk of the occurrence of a disease, information for determining whether a musculoskeletal disease has occurred in a subject and determining the possibility of its occurrence in a subject who has not a musculoskeletal disease at present and the prognosis thereof may be provided by measuring the marker. Accordingly, the term "diagnosis of a musculoskeletal disease" may also be expressed as "prediction of the risk of occurrence of a musculoskeletal disease".

In the present specification, the term "composition for diagnosing a musculoskeletal disease" refers to an integrated mixture or device including a means for measuring the expression level of the Zkscan8 (zinc finger protein with KRAB and SCAN domains 8) gene or a protein encoded by the gene for determining whether a musculoskeletal disease has occurred in a subject. Therefore, it may also be expressed as a "kit for diagnosing a musculoskeletal disease".

In a specific exemplary embodiment of the present disclosure, Zkscan8 (zinc finger protein with KRAB and SCAN domains 8) is a class of C2H2-ZNF (zinc finger) protein transcription factor. It is a transcription factor having a KRAB (Kruppel-associated box) domain and SRE-ZBP and SCAN (CTfin51, AW-1 and Number 18 cDNA) domains towards the N-terminal.

The Zkscan8 (zinc finger protein with KRAB and SCAN domains 8) gene may be represented by SEQ ID NO 1, which may be a base sequence registered in the NBCI with a gene symbol of Zkscan8 and a gene ID of 7745. Its optimized sequence may be represented by SEQ ID NO 3.

The protein encoded by the Zkscan8 gene may have an amino acid sequence registered with an NCBI accession number NP_001265048 and may be represented by SEQ ID NO 2.

In a specific exemplary embodiment of the present disclosure, musculoskeletal disease diagnosed by the composition of the present disclosure refers to a disease occurring in the nerves, muscles, nearby tissues, etc. of the neck, waist, and upper and lower limbs caused by workload on the musculoskeletal system (tendon, ligament, muscle, cartilage, nerve, bone, etc.). More specifically, the musculoskeletal disease may be one or more selected from a group consisting of a tendon disease, a ligament disease, a muscle disease, a cartilage disease, a nerve disease and a bone disease, specifically a tendon disease or a ligament disease, more specifically a tendon disease.

The tendon disease may be caused by chronic disorder or injury of the tendon, i.e., gradual wearing and tearing caused by overuse or aging, or acute tendon injury caused by the rupture of the tendon or separation from the bone.

The tendon disease may be one or more selected from a group consisting of tendon injury, tendon rupture, tendinitis, tendinosis and tendosynovitis.

Tendon rupture is a disease caused by partial tearing of the tendon, which is a fibrous tissue that attaches muscle to bone, or complete rupture into two pieces, and may be one or more selected from a group consisting of Achilles tendon rupture and patellar tendon rupture.

Tendinitis is a disease caused by the inflammation of the tendon itself due to the microrupture of the tendon occurring when abrupt and excessive load is applied to the musculotendinous unit, and may be one or more selected from a group consisting of Osgood-Schlatter disease, tenosynovitis, calcific tendinitis, patellar tendinitis, Achilles tendonitis, biceps tendinitis, rotator cuff tendinitis, lateral epicondylitis, supraspinatus tendinitis, triceps tendonitis and medial epicondylitis.

Tendinosis is a non-inflammatory or chronic inflammatory disease caused by degenerative changes in the collagen of the tendon itself owing to chronic overuse, and may be one or more selected from a group consisting of Achilles tendinopathy, patella tendinopathy and bicipital tendinopathy.

The ligament disease may be one or more selected from a group consisting of knee quadriceps ligament rupture, coronary ligament sprain, collateral ligament rupture, anterior cruciate ligament injury, posterior cruciate ligament injury, ankle ligament injury, ligament rupture and sprain.

The muscle disease is a disease caused by an injury induced by direct or indirect trauma, overload and overuse of muscle, and includes muscle loss, muscular dystrophy, muscle injury, muscle tone, muscle rupture, muscle wasting or muscle degeneration resulting therefrom. The muscle disease may be thigh muscle tone, muscular tissue injury, rotator cuff rupture, hamstring muscle injury, costoclavicular syndrome, knee quadriceps tendinosis, etc.

The cartilage disease is a disease caused by injury of cartilage, cartilage tissues and/or joint tissues (synovium, articular capsule, subchondral bone, etc.) due to mechanical stimulation or inflammatory responses, and includes a cartilage injury disease. The cartilage disease may be selected from a group consisting of degenerative arthritis, patella fracture, inflammatory arthritis, patella chondromalacia, osteochondromatosis, distortion, bursitis, meniscal injury, meniscal tear, meniscal cyst, intra-articular loose bodies, exfoliative osteochondritis, plica syndrome, genu varum, knock-knee and snapping knee.

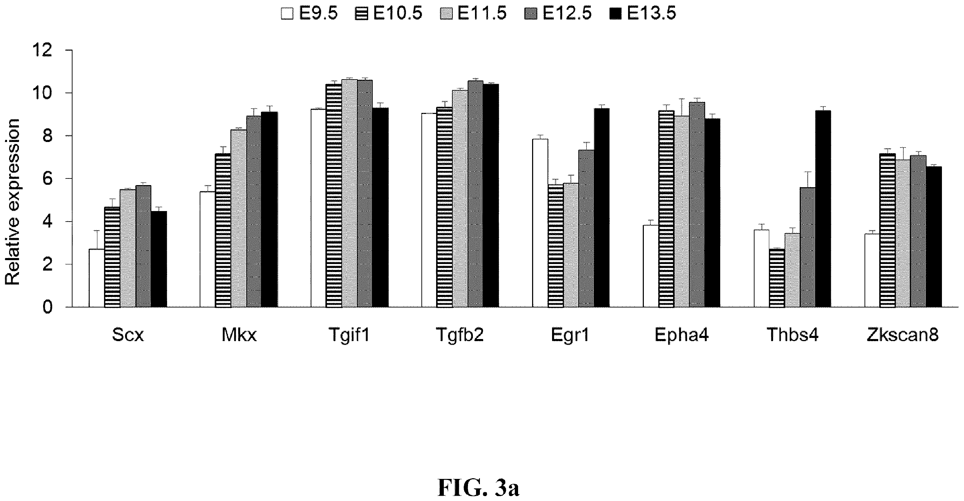

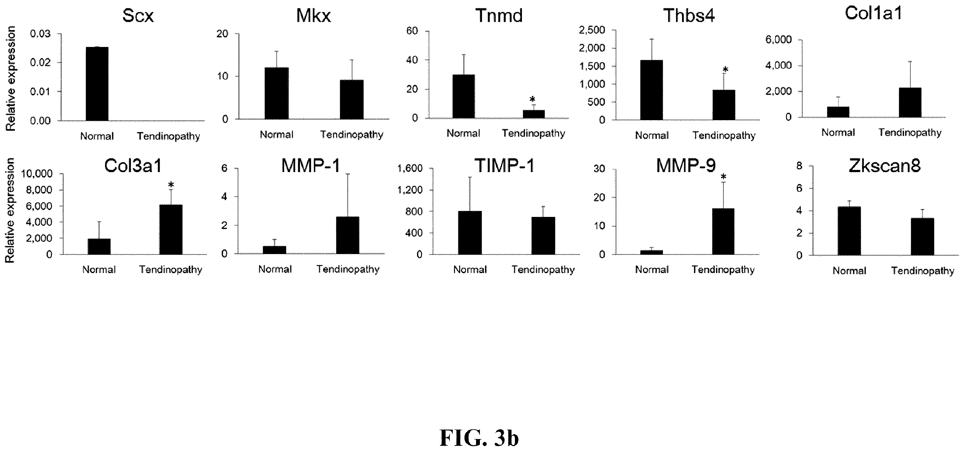

In a specific exemplary embodiment of the present disclosure, the musculoskeletal disease diagnosed by the composition of the present disclosure is a tendon disease. According to the present disclosure, it was confirmed from in-silico analysis that Zkscan8 (zinc finger protein with KRAB and SCAN domains 8), which is a biomarker presented by the present disclosure, is involved in the development and regeneration of the musculoskeletal system (tendon, ligament, muscle, bone, cartilage, nerve, etc.) and from the analysis of samples from an animal model of tendinosis that its expression is decreased as tendinosis proceeds (see FIG. 3). Accordingly, the Zkscan8 (zinc finger protein with KRAB and SCAN domains 8) of the present disclosure can provide information about the occurrence of a musculoskeletal disease.

In a specific exemplary embodiment of the present disclosure, the agent for measuring the expression level of the Zkscan8 gene is a nucleic acid molecule binding specifically to the Zkscan8 gene.

In the present specification, the term "nucleic acid molecule" encompasses DNA (gDNA and cDNA) and RNA molecules, and includes not only natural nucleotides, which are basic structural units, but also analogues having modified sugar or base moieties (Scheit, Nucleotide Analogs, John Wiley, New York (1980); Uhlman and Peyman, Chemical Reviews, 90: 543-584 (1990)). The "nucleic acid molecule binding specifically to the Zkscan8 gene" refers to a nucleic acid molecule which has a base sequence substantially complementary to the Zkscan8 gene of the present disclosure and can hybridize specifically thereto, and includes, for example, a primer and a probe.

In the present specification, the term "primer" refers to an oligonucleotide which acts as a starting point for synthesis under conditions that induce the synthesis of a primer extension product that is complementary to a nucleic acid chain (template), i.e., the presence of a nucleotide and a DNA polymerase and suitable temperature and pH. Specifically, the primer is a deoxyribonucleotide single chain. The primer used in the present disclosure may include naturally occurring dNMPs (i.e., dAMP, dGMP, dCMP and dTMP), modified nucleotides or non-naturally occurring nucleotides and ribonucleotides.

The primer of the present disclosure may be an extension primer that is annealed to a target nucleic acid and forms a sequence complementary to the target nucleic acid by a template-dependent nucleic acid polymerase. It is extended to a position where an immobilization probe is annealed and occupies the area where it is annealed.

The extension primer used in the present disclosure includes a hybridization nucleotide sequence complementary to a specific base sequence of a target nucleic acid, e.g., the Zkscan8 gene. The term "complementary" means that a primer or a probe is sufficiently complementary to hybridize selectively to a target nucleic acid sequence under certain annealing or hybridization conditions. The term encompasses both substantially complementary and perfectly complementary. Specifically, it means perfectly complementary. In the present specification, the term "substantially complementary sequence" is meant to include not only a completely matching sequence but also a sequence partially mismatching with a specific sequence to be compared within a range where it can anneal to the sequence and serve as a primer.

In the present disclosure, the primer should be long enough to prime the synthesis of an extension product in the presence of a polymerase. The suitable length of the primer is determined by a number of factors, such as temperature, pH and the source of the primer, but is typically 15-30 nucleotides. Short primer molecules generally require lower temperatures to form a sufficiently stable hybrid complex with a template. The design of such a primer can be easily carried out by those skilled in the art with reference to a target nucleotide sequence using, for example, a program for primer design (e.g., PRIMER 3).

In the present specification, the term "probe" refers to a natural or modified monomer or a linear oligomer, which includes a deoxyribonucleotide and a ribonucleotide that can be hybridized with a specific nucleotide sequence. Specifically, the probe is single-stranded for maximum efficiency in hybridization. More specifically, it is a deoxyribonucleotide. As the probe used in the present disclosure, not only a sequence perfectly complementary to a specific base sequence of the Zkscan8 gene but also a sequence substantially complementary thereto may be used as long as specific hybridization is not interrupted. Because the stability of a duplex formed by hybridization is determined by the mismatch of sequences at terminals in general, it is preferred to use a probe which is complementary to the 3'- end or 5'-end of the target sequence.

Conditions suitable for the hybridization may be determined with reference to Joseph Sambrook, et al., Molecular Cloning, A Laboratory Manual, Cold Spring Harbor Laboratory Press, N.Y. (2001) and Haymes, B. D., et al., Nucleic Acid Hybridization, A Practical Approach, IRL Press, Washington, D.C. (1985).

In a specific exemplary embodiment of the present disclosure, the agent for measuring the expression level of a protein encoded by the Zkscan8 gene is an antibody or an aptamer binding specifically to the protein encoded by the Zkscan8 gene.

In the present specification, the term "antibody" refers to specific protein molecule directed to an antigenic site. For the purpose of the present disclosure, the antibody refers to an antibody that can bind specifically to a protein encoded by the Zkscan8 gene, and may include all antibodies such as a polyclonal antibody, monoclonal antibody, a recombinant antibody, etc. In addition, a special antibody such as a humanized antibody, etc. may also be included. The antibody may include a complete form having two full-length light chains and two full-length heavy chains as well as a functional fragment of the antibody molecule. The functional fragment of the antibody molecule refers to a fragment retaining at least its antigen-binding function, and may include Fab, F(ab'), F(ab')₂, Fv, etc. As the antibody, any one prepared by those having ordinary knowledge in the art using known technology may be used.

In the present specification, the term "aptamer" refers to a special type of polynucleotide consisting of a single-stranded nucleic acid (DNA, RNA or modified nucleic acid) that has a stable three-dimensional structure on its own and can bind to a target molecule with high affinity and specificity. Since the aptamer is composed of a polynucleotide which has higher stability than a protein, has a simple structure and can be synthesized easily, while it can bind specifically to an antigenic material like an antibody, it can be used instead of an antibody.

The general contents about the aptamer are described in detail in Bock LC et al., Nature 355 (6360): 5646 (1992), Hoppe-Seyler F, Butz K "Peptide aptamers: powerful new tools for molecular medicine". J Mol Med. 78 (8): 42630 (2000) and Cohen BA, Colas P, Brent R. "An artificial cell-cycle inhibitor isolated from a combinatorial library". Proc Natl Acad Sci USA. 95 (24): 142727 (1998).

In a specific exemplary embodiment of the present disclosure, the diagnostic sample of the present disclosure may be one or more body fluid selected from a group consisting of urine, saliva, semen, tears, amniotic fluid, cerebrospinal fluid, synovial fluid, pericardial fluid, peritoneal fluid, blood, plasma and serum, or a tissue or a cell isolated from the musculoskeletal system. Most specifically, it may be a tissue or a cell isolated from the musculoskeletal system.

According to the present disclosure, a musculoskeletal disease may be diagnosed by measuring the concentration of the Zkscan8 of the present disclosure. Through this, patients' satisfaction and simplicity and economy of procedures can be achieved by providing highly reliable clinical information through biopsy.

In another aspect of the present disclosure, the present disclosure provides a method for providing information necessary for diagnosing a musculoskeletal disease, which includes:
1) a step of measuring the expression level of a Zkscan8 (zinc finger protein with KRAB and SCAN domains 8) gene or a protein encoded by the gene in a biological sample isolated from a subject suspected of a musculoskeletal disease; and
2) a step of comparing the measured expression level of the Zkscan8 gene or the protein encoded by the gene with the expression level of a normal control group sample.

The description about the musculoskeletal disease to be diagnosed in the present disclosure will be omitted to avoid unnecessary redundancy because it was described in detail above.

In the present disclosure, the term "biological sample" refers to any sample obtained from a mammal including human, which contains a cell expressing Zkscan8, and includes but is not limited to a tissue, an organ, a cell or a cell culture. More specifically, the cell expressing Zkscan8 is a tissue or a cell constituting the musculoskeletal system, most specifically a tendon tissue, a tendon cell, a ligament tissue or a ligament cell.

The expression level of the Zkscan8 gene or the protein encoded thereby may be measured using a primer, a probe, an antibody or an aptamer specific for Zkscan8.

The step of measuring the expression level of Zkscan8 in a biological sample isolated from a subject suspected of a musculoskeletal disease may be performed by various expression level measurement methods known in the art such as hybridization, immunoassay or gene amplification, although not being specially limited thereto. As a result of the measurement, if the expression level of Zkscan8 has been decreased, information necessary for diagnosing and determining that a musculoskeletal disease has occurred in the subject may be provided.

In the present disclosure, the subject suspected of a musculoskeletal disease refers to a subject who already has or is predicted to have a musculoskeletal disease.

Also, in the present disclosure, the "normal control group" refers to a subject not having the musculoskeletal disease to be detected.

In the present specification, the term "decreased of the expression level" means that the expression of Zkscan8 is significantly decreased due to a musculoskeletal disease.

In another aspect of the present disclosure, the present disclosure provides a composition for preventing or treating a musculoskeletal disease, which contains a Zkscan8 (zinc finger protein with KRAB and SCAN domains 8) protein as an active ingredient.

In another aspect of the present disclosure, the present disclosure provides a pharmaceutical composition for preventing and treating a musculoskeletal disease, which contains a vector including a Zkscan8 (zinc finger protein with KRAB and SCAN domains 8) gene, a cell including the vector or a culture thereof as an active ingredient.

The present disclosure may relate to a novel use of the Zkscan8 protein for preventing or treating a musculoskeletal disease.

The Zkscan8 (zinc finger protein with KRAB and SCAN domains 8) gene may be represented by SEQ ID NO 1, which may be a base sequence registered in the NBCI with a gene symbol of Zkscan8 and a gene ID of 7745. The protein encoded by the Zkscan8 gene may have an amino acid sequence registered with an NCBI accession number NP_001265048 and may be represented by SEQ ID NO 2. The optimized sequence of the Zkscan8 gene may be represented by SEQ ID NO 3.

In an example of the present disclosure, it was confirmed that, when rat with tendon injury was treated with Zkscan8-overexpressed cells, abnormal behavior pattern caused by tendon tissue injury was decreased and recovery of tendon tissue was promoted as compared to an untreated rat. In addition, a series of compensatory responses such as recovery of collage fiber coherence, etc. for attenuating the pathological processes of a musculoskeletal disease was observed as the expression of Zkscan8 was increased. Accordingly, a composition containing the Zkscan8 gene or protein may exhibit an effect of preventing or treating a musculoskeletal disease.

In the present disclosure, the Zkscan8 protein may include a variant or a functional equivalent thereof. The variant or functional equivalent of the protein refers to a polypeptide which has a sequence homology of 60%, specifically 70%, more specifically 80%, or higher to the amino acid sequence of the Zkscan8 protein as a result of the addition, substitution or deletion of amino acids, and exhibits substantially the same activity as the Zkscan8 protein of the present disclosure. The functional equivalent may include, for example, an amino acid sequence variant with some of the amino acids in the amino acid sequence of the Zkscan8 protein being substituted, deleted or added. The substitution of amino acids may be specifically conservative substitution, and examples of naturally occurring conservative substitution of amino acids include: aliphatic amino acids (Gly, Ala, Pro), hydrophobic amino acids (Ile, Leu, Val), aromatic amino acids (Phe, Tyr, Trp), acidic amino acids (Asp, Glu), basic amino acids (His, Lys, Arg, Gln, Asn) and sulfur-containing amino acids (Cys, Met). The deletion of amino acids may be specifically located at the regions not directly involved in the activity of the Zkscan8 protein of the present disclosure. In addition, a polypeptide derivative whose chemical structure has been changed partially while retaining the basic structure and physiological activity of the Zkscan8 protein may be encompassed in the scope of the functional equivalent. For example, a fusion protein, etc. prepared from fusion with another protein in order to change the stability, storage property, volatility, solubility, etc. of the Zkscan8 protein of the present disclosure while retaining the basic structure and physiological activity may be included.

The Zkscan8 protein according to the present disclosure may be used as it is or in the form of a pharmaceutically acceptable salt. In the present disclosure, "pharmaceutically acceptable" means that the protein is physiologically and does not normally cause allergic reactions such as gastrointestinal disorder or dizziness or similar responses when administered to human without inhibiting the action of the active ingredient. The salt may be specifically an acid addition salt formed from a pharmaceutically acceptable free acid. As the free acid, an organic acid or an inorganic acid. The organic acid includes but is not limited to citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, glutamic acid and aspartic acid. And, the inorganic acid includes but is not limited to hydrochloric acid, bromic acid, sulfuric acid and phosphoric acid.

In the present disclosure, the Zkscan8 protein or a fragment thereof may be extracted from the nature, synthesized (Merrifield, J. Amer. chem. Soc. 85: 2149-2156, 1963) or prepared by gene recombination based on a DNA sequence Sambrook et al, Molecular Cloning, Cold Spring Harbor Laboratory Press, New York, USA, 2nd edition, 1989).

In the present disclosure, the vector may be one or more selected from a group consisting of a linear DNA, a plasmid DNA and a recombinant viral vector, and the virus may be one or more selected from a group consisting of retrovirus, adenovirus, adeno-associated virus, herpes simplex virus and lentivirus.

Methods for delivering the vector of the present disclosure into a host cell includes, for example, microinjection (Harland and Weintraub, J. Cell Biol. 101: 1094-1099 (1985)), calcium phosphate precipitation (Chen and Okayama, Mol. Cell. Biol. 7: 2745-2752 (1987)), electroporation (Tur-Kaspa et al., Mol. Cell Biol., 6: 716-718 (1986)), liposome-mediated transfection (Nicolau et al., Methods Enzymol., 149: 157-176 (1987)), DEAE-dextran treatment (Gopal, Mol. Cell Biol., 5: 1188-1190 (1985)) and gene bombardment (Yang et al., Proc. Natl. Acad. Sci., 87: 9568-9572 (1990)), although not being limited thereto.

The cell including the vector may be one or more selected from a group consisting of a stem cell, a dendritic cell, an autologous tumor cell and an established tumor cell, although not being specially limited thereto.

In the present disclosure, the term "stem cell" refers to an undifferentiated cell having the ability to differentiate into various body tissues. The stem cells can be classified into totipotent stem cells, pluripotent stem cells, multipotent stem cells, etc. The term stem cell may be used interchangeably with such terms as precursor cell, progenitor cell, etc. In the present disclosure, the stem cell may be an embryonic stem cell (ESC), an induced pluripotent stem cell (iPSC) or a mesenchymal stem cell (MSC), more specifically a mesenchymal stem cell.

The mesenchymal stem cell may be a mesenchymal stem cell derived from the bone marrow, fat, umbilical cord or umbilical cord blood. Specifically, the mesenchymal stem cell may be an umbilical cord-derived mesenchymal stem cell.

The description about the musculoskeletal disease to be diagnosed or treated in the present disclosure will be omitted to avoid unnecessary redundancy because it was described in detail above.

In the present specification, the term "prevention" refers to prevention of the occurrence of a disease or a disorder in a subject who has not been diagnosed with the disease or disorder but has the possibility of having the disease or disorder.

In the present specification, the term "treatment" refers to: (a) inhibition of the development of a disease, a disorder or symptoms; (b) alleviation of disease, a disorder or symptoms; or (c) removal of a disease, a disorder or symptoms. When the composition of the present disclosure is administered to a subject, it inhibits the development of the symptoms of a musculoskeletal disease or removes or alleviates them by promoting or activating the expression of Zkscan8, thereby promoting differentiation into the musculoskeletal system and inducing recovery of musculoskeletal tissues and formation of collagen. Accordingly, the composition of the present disclosure may be used as a composition for treating a musculoskeletal disease on its own or may be used as a therapeutic adjuvant for the disease as being administered together with another pharmacological ingredient. Accordingly, in the present specification, the term "treatment" or "therapeutic agent" encompasses "therapeutic support" or "therapeutic adjuvant".

In the present specification, the term "administration" or "administer" refers to administration of a therapeutically effective amount of the composition of the present disclosure directly into a subject.

In the present disclosure, the term "therapeutically effective amount" refers to the content of the composition of the present disclosure containing the active ingredient in an amount enough to provide a therapeutic or prophylactic effect in a subject. Therefore, the term includes the meaning of "prophylactically effective amount".

In the present specification, the term "subject" includes human, mouse, rat, guinea pig, dog, cat, horse, cow, pig, monkey, chimpanzee, baboon or rhesus monkey without limitation. Specifically, the subject of the present disclosure is human.

In an example of the present disclosure, an expression vector was prepared by inserting the Zkscan8 (zinc finger protein with KRAB and SCAN domains 8) gene of SEQ ID NO 1 into a pscAAV vector. It was named 'pscAAV-Zkscan8' and its cleavage map is shown in FIG. 1b. The prepared expression vector was transfected into human umbilical cord-derived mesenchymal stem cells (UCMSCs). Treatment with the Zkscan8-overexpressed umbilical cord-derived mesenchymal stem cells increased the ultimate failure load and stiffness of tendon. In addition, the treatment with the Zkscan8-overexpressed umbilical cord-derived mesenchymal stem cells exhibited the effect of inhibiting inflammation of tendon tissues, preventing degenerative changes, recovering collage fiber coherence and inhibiting heterotopic cartilage formation. That is to say, it can be seen that, when the Zkscan8-overexpressed umbilical cord-derived mesenchymal stem cells were applied to full-thickness rupture injury, they can regenerate the defective tissues of the injured tendon and recover and improve the function of the tendon. Accordingly, the composition of the present disclosure for preventing or treating a musculoskeletal disease increases the expression or activity of Zkscan8.

In another aspect of the present disclosure, a method for screening a composition for preventing or treating a musculoskeletal disease, which includes:
(a) a step of contacting injured tendon, ligament, muscle, cartilage or bone cells with a test substance; and
(b) a step of measuring the expression level of a Zkscan8 (zinc finger protein with KRAB and SCAN domains 8) gene or protein in the sample,

If the expression level of the Zkscan8 is increased as compared to a normal control group in the step (b), the test substance is determined as a composition for preventing or treating a musculoskeletal disease.

The description about the musculoskeletal disease to be prevented or treated in the present disclosure will be omitted to avoid unnecessary redundancy because it was described in detail above.

In the present disclosure, the term "injured tendon, ligament, muscle, cartilage, bone or nerve cells" refer to any sample containing musculoskeletal cells with decreased expression of Zkscan8, obtained from mammals including human. The injured tendon, ligament, muscle, cartilage, bone or nerve cells may be obtained from musculoskeletal tissues or organs injured by external pressure, operation, rupture, tearing, fracture, inflammation, etc., and include cells or a cell culture. More specifically, they may refer to injured tendon, ligament, muscle, cartilage, bone or nerve cells, specifically tendon cells or ligament cells.

The term "test substance" used with reference to the screening method of the present disclosure refers to an unknown substance added to a sample containing cells expressing Zkscan8 in order to test if it affects the expression level of Zkscan8. The test substance includes a compound, a nucleotide, a peptide and a natural extract, although not being limited thereto. The step of measuring the expression level of Zkscan8 in the biological sample treated with the test substance may be performed by various expression level measurement methods known in the art. If the expression level of Zkscan8 is increased, the test substance may be determined as a composition for preventing or treating a musculoskeletal disease.

In the present specification, the term "increase of the expression level" means that the expression level or intrinsic function of the Zkscan8 gene is significantly increased by the test substance to such a level that the recovery of injured musculoskeletal tissues is detectable.

### [Advantageous Effects]

The features and advantages of the present disclosure may be summarized as follows:
(a) The present disclosure provides a composition for diagnosing a musculoskeletal disease, a composition for preventing or treating a musculoskeletal disease and a method for screening the same.
(b) The Zkscan8 of the present disclosure can be effectively used as an excellent biomarker for obtaining accurate information about the occurrence and progression stages of a musculoskeletal disease, specifically a tendon disease or a ligament disease.
(c) The composition of the present disclosure can be effectively applied as a composition for preventing or treating a musculoskeletal disease through Zkscan8 overexpression.

### [Brief Description of Drawings]

FIG. 1a shows a model for the structure of the ZSCAN family.
FIG. 1b shows the cleavage map of pscAAV-Zkscan8.
FIG. 1c schematically shows the structure of a pscAAV-GFP vector and a pscAAV-Zkscan8 vector.
FIG. 2 summarizes the distribution of datasets during embryogenesis (stages E9.5-E15.5).
FIG. 3a shows a result of analyzing the expression pattern of tenogenesis markers and Zkscan8 depending on the stages of embryogenesis.
FIG. 3b shows a result of measuring the expression pattern of tenogenesis markers and Zkscan8 in a normal human tendon (Normal) and the tendon of a patient with rotator cuff disease (Tendinopathy).
FIG. 4a shows the microscopic images of Zkscan8-introduced umbilical cord-derived mesenchymal stem cells (MSC-Zk8) and a control group (MSC-GFP).
FIG. 4b shows a result of quantifying the proliferation ability of Zkscan8-introduced umbilical cord-derived mesenchymal stem cells (MSC-Zk8) and a control group (MSC-GFP).
FIG. 4c shows a result of measuring the expression of the Zkscan8 gene in Zkscan8-introduced umbilical cord-derived mesenchymal stem cells (MSC-Zk8) and a control group (MSC-GFP) by RT-PCR.
FIG. 4d shows a result of analyzing the expression of the Zkscan8 protein in Zkscan8-introduced umbilical cord-derived mesenchymal stem cells (MSC-Zk8) and a control group (MSC-GFP) by western blotting.
FIG. 4e shows a result of quantifying the expression of the Zkscan8 protein in Zkscan8-introduced umbilical cord-derived mesenchymal stem cells (MSC-Zk8) and a control group (MSC-GFP) by western blotting.
FIGS. 5a-5d show a result of evaluating the effect of Zkscan8 overexpression on tendon differentiation.

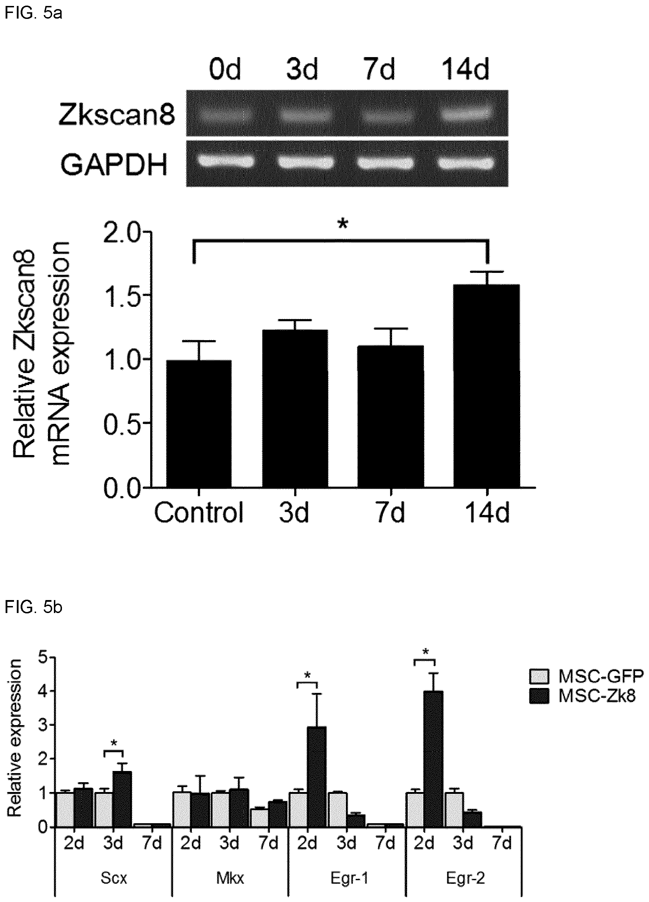

FIG. 5a shows a result of culturing umbilical cord-derived mesenchymal stem cells (UCMSCs) in a tendon differentiation medium for 2 weeks and then quantifying the expression level of Zkscan8 over time by RT-PCR.
FIG. 5b shows a result of culturing Zkscan8-introduced umbilical cord-derived mesenchymal stem cells (MSC-Zk8) and a control group (MSC-GFP) for 7 days and then analyzing the expression level of tenogenic transcription factors (Scx, Mkx, Egr-1, Egr-2) over time by qRT-PCR.

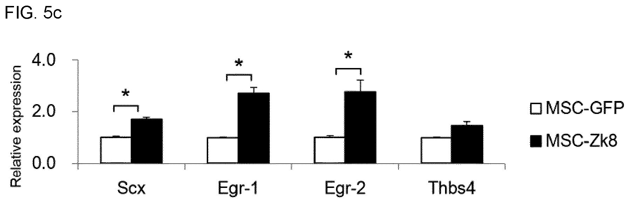

FIG. 5c shows a result of culturing Zkscan8-introduced umbilical cord-derived mesenchymal stem cells (MSC-Zk8) and a control group (MSC-GFP) in a tendon differentiation medium for 3 days through 3D culture and then analyzing the expression level of tendon-related markers (Scx, Egr-1, Egr-2, Thbs4) by qRT-PCR.

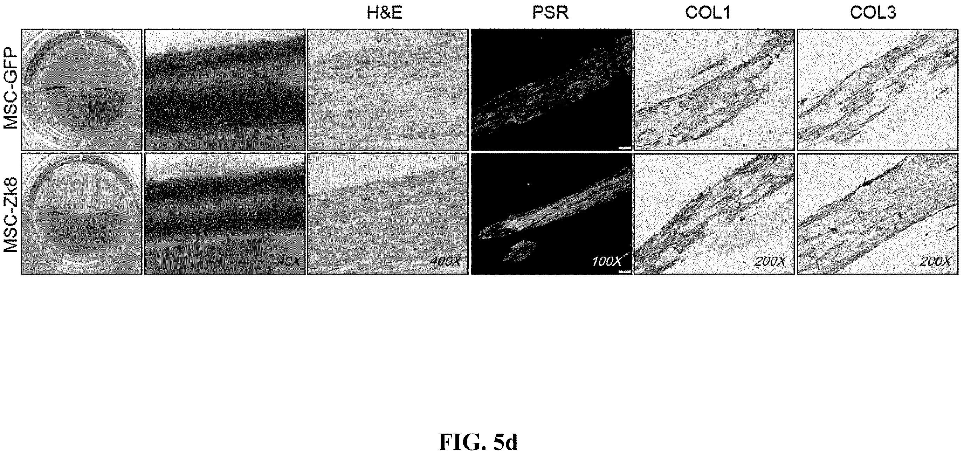

FIG. 5d shows a result of analyzing the histological characteristics of Zkscan8-introduced umbilical cord-derived mesenchymal stem cells (MSC-Zk8) and a control group (MSC-GFP) depending on the induction of tendon differentiation when they were cultured in a tendon differentiation medium for 3 days through 3D culture.

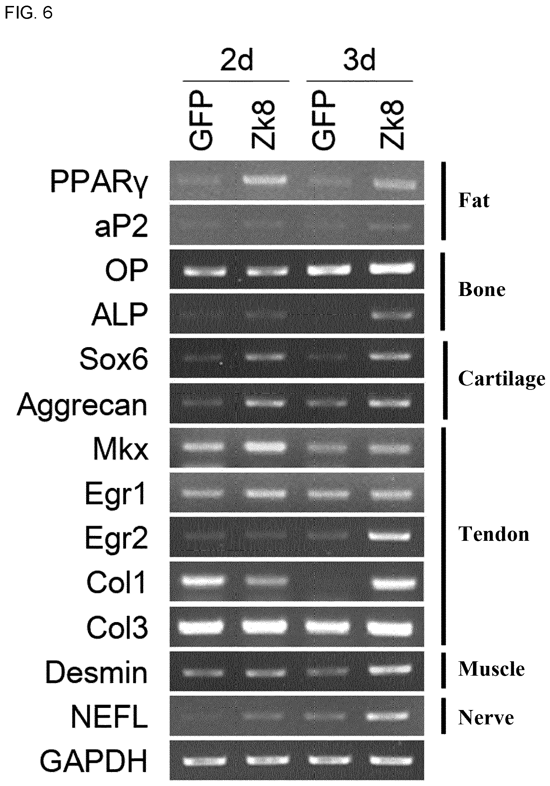

FIG. 6 shows a result of analyzing the expression pattern of fat, bone, cartilage, tendon, muscle and nerve cell-related markers in Zkscan8-introduced umbilical cord-derived mesenchymal stem cells (MSC-Zk8) and a control group (MSC-GFP).

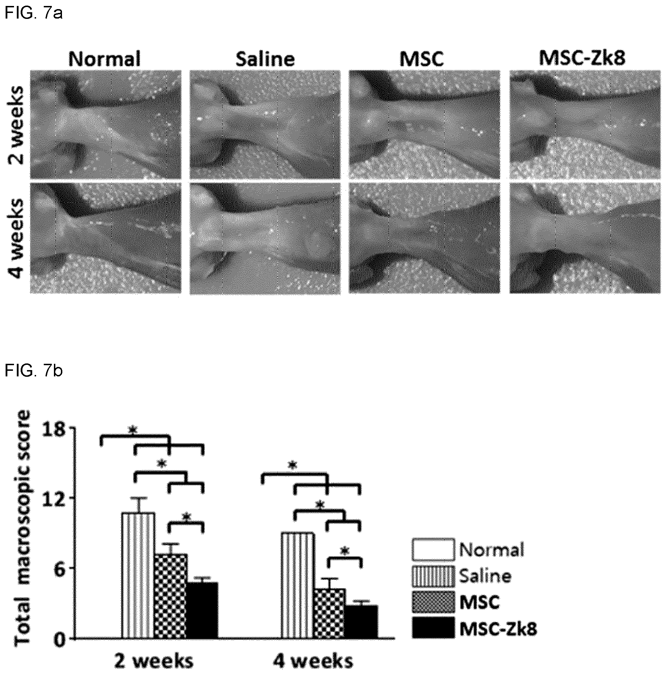

FIG. 7a shows the macroscopic images of the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment.
FIG. 7b shows a result of analyzing total macroscopic score for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).

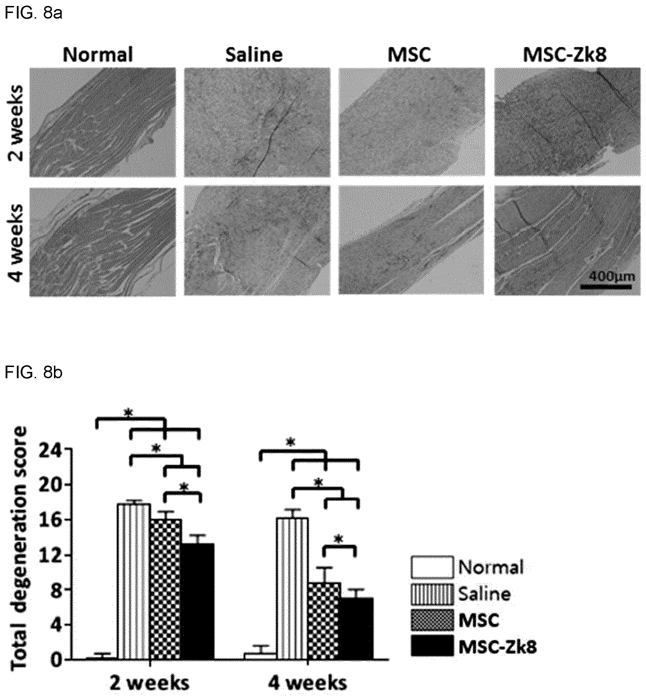

FIG. 8a shows the H&E staining images of the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment (magnification: x100).
FIG. 8b shows a result of analyzing total degeneration score for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).

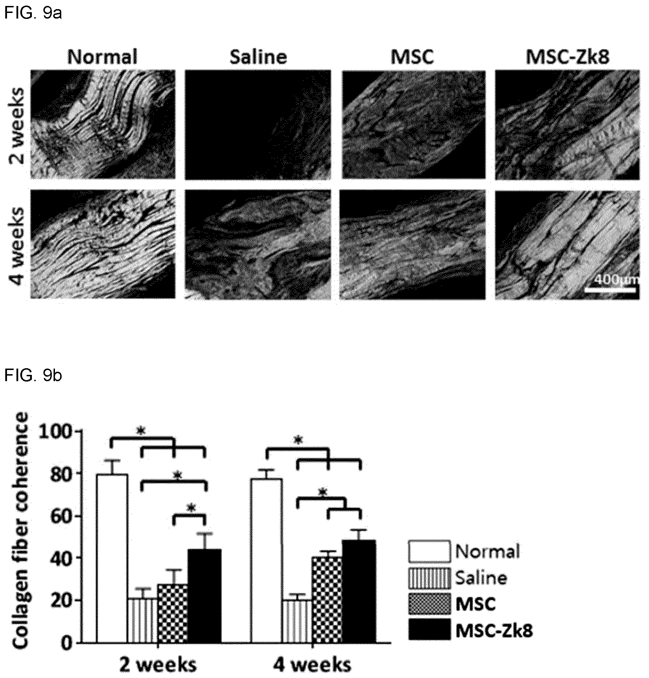

FIG. 9a shows the PSR staining images of the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment (magnification: x100).
FIG. 9b shows a result of analyzing collage fiber coherence for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).

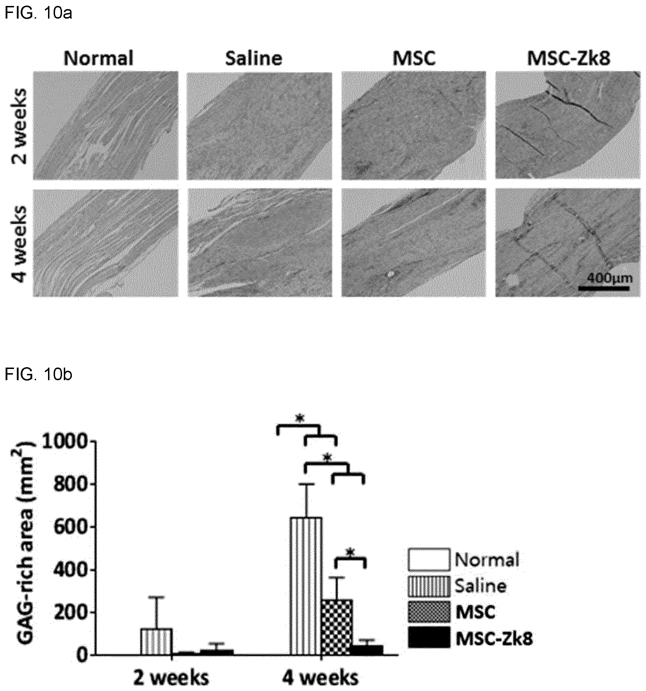

FIG. 10a shows the Saf-O staining images of the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment (magnification: x100).
FIG. 10b shows a result of analyzing glycoaminoglycan (GAG)-rich area for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).

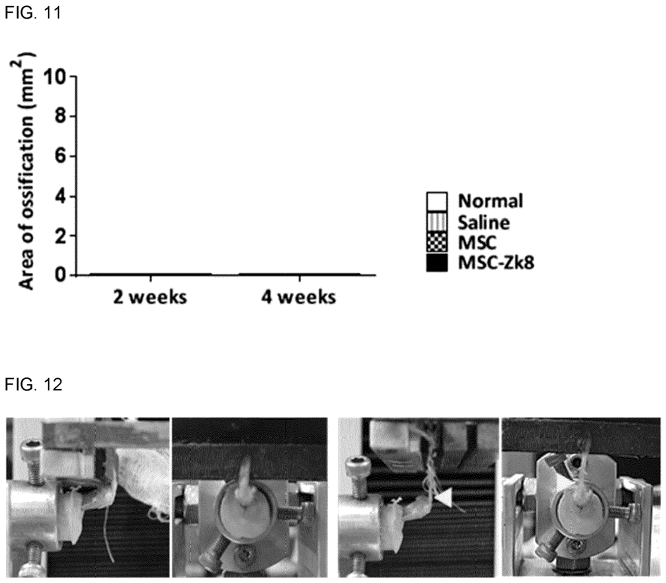

FIG. 11 shows a result of analyzing area of ossification for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).
FIG. 12 shows sampling from a normal group (Normal), a physiological saline group (Saline), an umbilical cord-derived mesenchymal stem cell group (MSC) and a Zkscan8-introduced umbilical cord-derived mesenchymal stem cell group (MSC-Zk8) and the procedure of a biomechanical test.

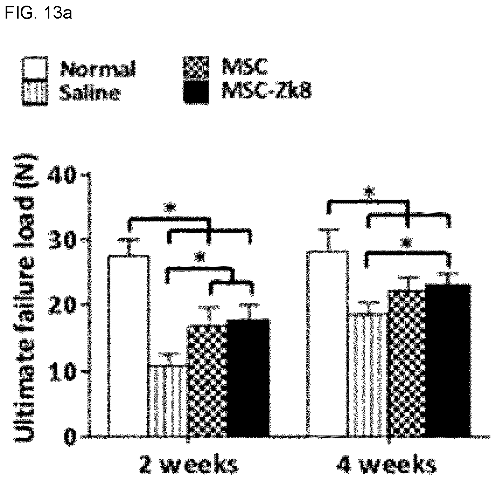

FIG. 13a shows a result of analyzing ultimate failure load for the regenerated tendon taken from a normal group (Normal), a physiological saline group (Saline), an umbilical cord-derived mesenchymal stem cell group (MSC) and a Zkscan8-introduced umbilical cord-derived mesenchymal stem cell group (MSC-Zk8). All quantitative data are presented as mean ± standard error (S.E.M) (*p < 0.05).

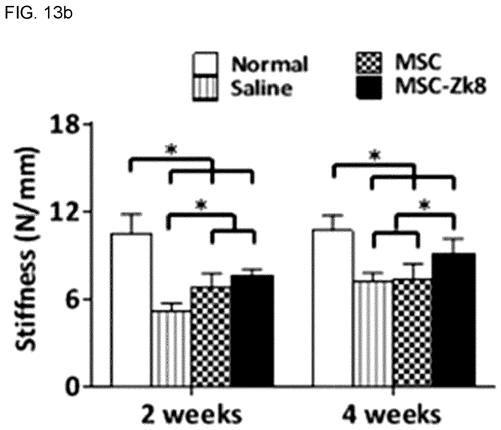

FIG. 13b shows a result of analyzing stiffness for the regenerated tendon taken from a normal group (Normal), a physiological saline group (Saline), an umbilical cord-derived mesenchymal stem cell group (MSC) and a Zkscan8-introduced umbilical cord-derived mesenchymal stem cell group (MSC-Zk8). All quantitative data are presented as mean ± standard error (S.E.M) (*p < 0.05).

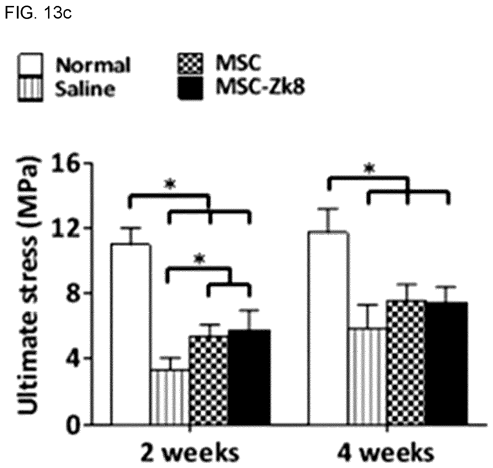

FIG. 13c shows a result of analyzing ultimate stress for the regenerated tendon taken from a normal group (Normal), a physiological saline group (Saline), an umbilical cord-derived mesenchymal stem cell group (MSC) and a Zkscan8-introduced umbilical cord-derived mesenchymal stem cell group (MSC-Zk8). All quantitative data are presented as mean ± standard error (S.E.M) (*p < 0.05).

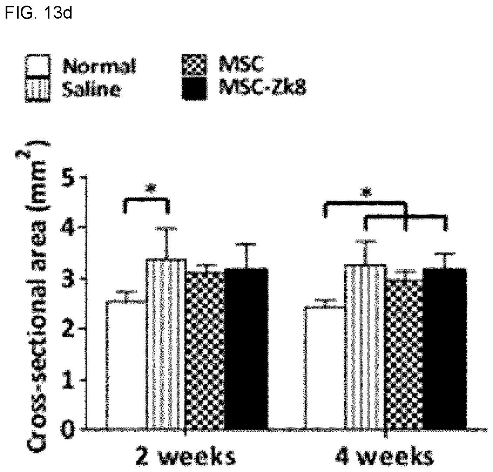

FIG. 13d shows a result of analyzing cross-sectional area for the regenerated tendon taken from a normal group (Normal), a physiological saline group (Saline), an umbilical cord-derived mesenchymal stem cell group (MSC) and a Zkscan8-introduced umbilical cord-derived mesenchymal stem cell group (MSC-Zk8). All quantitative data are presented as mean ± standard error (S.E.M) (*p < 0.05).

### [Best Mode]

Hereinafter, the present disclosure will be described in more detail through examples. The examples are provided only to describe the present disclosure more specifically and it will be obvious to those having ordinary knowledge in the art that the scope of the present disclosure is not limited by the examples.

### Examples

### Experimental methods

### Screening and selection of genes

In order to search for biomarkers related with musculoskeletal diseases, whole transcriptome expression profiling microarray datasets GSE30138 and GSE54207 and RNA sequencing dataset GSE65180 associated with mouse limb tenogenesis were downloaded from the Gene Expression Omnibus (GEO) database (http://www.ncbi.nlm.gov/geo/) of the National Center for Biotechnology Information (NCBI) and analyzed for screening of tendon regeneration candidate genes.

For GSE30138 and GSE54207, the GPL1261 platform (Affymetrix mouse genome 430 2.0 array) was used. And, for GSE65180, the GPL13112 platform [Illumina Hiseq 2000 (*Mus musculus*)] was used. Genes showing similar expression patterns depending on embryonic stages as tenogenesis markers (Tgf-β2, Scx, Mkx, Egr1, Tnmd, Thbs4, Col1, Gag, Tnc), myogenesis markers (Pax3, Pax7, Myf5, Des, MyoD, myogenin, MRF4, myostatin, MHC2), osteogenesis marker (Runx2, Osterix, Alp, Ocn, Opn) and chondrogenesis markers (Comp, Sox9, Chad, Acan, Col2a1) were screened primarily.

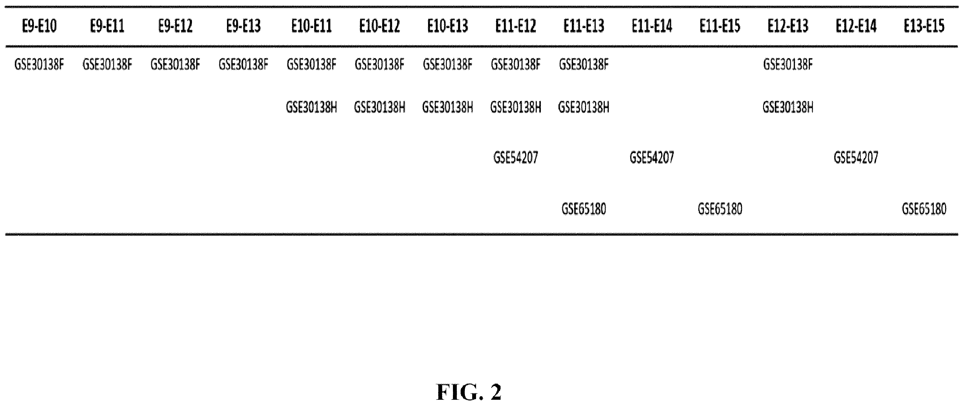

For construction of the protein-protein interaction (PPI) network and investigation of the interaction between genes, analysis was conducted using the TiCoNE and ClueGo plugins of the Cytoscape software (http://www.cytoscape.org/) (FIG. 2). Next, in order to screen hub genes, significant modules closely related in the PPI network such as molecular complex detection (MCODE; degree cutoff = 2.1, node score cutoff = 0.2, k-core = 2, max. depth = 100) and Markov clustering (MCL) were used. Candidate genes were screened by analyzing the relationship of the screened clusters with tendon regeneration through gene ontology (GO) analysis.

### RNA-seq analysis

Total RNAs of a normal tendon (n = 5) and a rotator cuff (n = 6) were extracted using an RNeasy Plus universal kit (Cat. 73404. Qiagen, USA) according to the manufacturer's protocol. The integrity of the total RNAs was identified with an Agilent 2100 bioanalyzer (Agilent Technologies, Santa Clara, CA, USA) and a cDNA library was created using a TruSeq stranded mRNA library prep kit (Cat. 20020594; Illumina, USA) according to the manufacturer's protocol. The pooled library was loaded on HiSeq 2000 at a concentration of 2 nM and then sequenced (v3 SBS chemistry, 76 cycles, paired-end).

### Culturing of human umbilical cord-derived stem cells and tendon cells

All the tissues used in the study were acquired and used under the consent of patients. Umbilical cord and tendon tissues were washed 2-3 times with Ca²⁺- and Mg²⁺-free Dulbecco's phosphate-buffered saline (DPBS, GIBCO, NY, USA) supplemented with antibiotics (100 U/mL penicillin, 100 µg/mL streptomycin sulfate and 0.25 µg/mL amphotericin B (antibiotic-antimycotic solution; Welgene, Daegu, Korea)) in order to remove impurities such as blood, etc.

Umbilical cord was acquired from a patient who received Caesarean section. After measuring length and weight, the umbilical cord was cut to about 2-4 mm in length using surgical scissors, and an amount corresponding to 1 g was aligned and inoculated onto a 150 cm² culture dish. After the umbilical cord completely adhered to the culture dish, it was cultured using a culture medium (LG DMEM, 10% fetal bovine serum (FBS; Welgene, Daegu, Korea) and antibiotic-antimycotic solution) at 37 °C while supplying 5% CO².

Tendon tissues obtained from a patient with complete rotator cuff tear were cultured at 37 °C for 2 hours with light shaking in HG DMEM (high-glucose Dulbecco's modified Eagle's medium, Welgene, Daegu, Korea) supplemented with 0.3% type 2 collagenase (GIBCO) and antibiotics. Subsequently, after adding a culture medium (HG DMEM, 10% FBS) and antibiotics (antibiotic-antimycotic solution) of the same volume, undissociated tissues were removed using a 100-µm cell filter. Cells recovered by centrifuging (500 g, 15 minutes) at 20 °C were washed twice with a culture medium. The recovered cells were counted by trypan blue exclusion assay. The cells were transferred onto a culture dish at a density of 2×10⁴ to 5×10⁴ cells/cm² and then cultured at 37 °C in a 5% CO² incubator.

When the cells grew to fill about 60-80% of the culture dish, they were washed twice with DPBS and adherent cells were detached by treating with trypsin-EDTA (Welgene, Daegu, Korea) containing 0.05% trypsin and 0.53 mM EDTA (ethylenediaminetetraacetic acid) for 3 minutes. Then, the cells were stained and counted by trypan blue exclusion assay. Human umbilical cord-derived stem cells (3,333 cells/cm²) and tendon cells were cultured continuously at a ratio of 1:3.

### Preparation of Zkscan8-introduced umbilical cord-derived mesenchymal stem cells (MSC-Zk8)

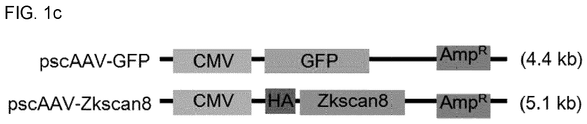

A pscAAV-GFP vector plasmid (Cell Biolabs, CA, USA) was used as a control group vector. After cleaving the GFP region with restriction enzymes BamHI and Sall, Zkscan8 was cloned into the region using primers (FP: 5'-AAGGATCCATGTACCCATACGATGTTCCAGATTACGCTATGGCGGAGGAAAGTC GG-3', RP: 5'-AAGTCGACCTAGACTGAGATAGACTC-3') and BamHI and Sall restriction enzymes. The cleavage map of the pscAAV-Zkscan8 vector is shown in FIG. 1b, and the structures of the prepared pscAAV-GFP and pscAAV-Zkscan8 vectors are schematically shown in FIG. 1c. The sequence of pscAAV-Zkscan8 was analyzed by sequencing. For production of viral packaging stocks, three vectors (target expression vectors, pAAV-RC, pHelper) were introduced into 293 cells according to the manufacturer's instructions. 72 hours later, the culture medium including the cells were collected, frozen and then thawed. An adenovirus including the Zkscan8 gene was obtained by repeating this procedure. The prepared virus was used as a Zkscan8 gene delivery system for human umbilical cord-derived stem cells.

### Analysis of proliferation ability

Proliferation ability was analyzed by measuring the amount of living cells by WST assay. After inoculating the gene-introduced umbilical cord-derived mesenchymal stem cells onto a culture dish at a density of 5×10⁴ cells/cm², 10 µL of Ez-Cytox (DoGEN, Guro, Korea) was added to the culture medium on days 3, 5, 9, 12 and 15. After incubation at 37 °C in a 5% CO² incubator for 1 hour, absorbance was measured at a wavelength of 450 nm.

### Differentiation of umbilical cord-derived mesenchymal stem cells into tendon

The umbilical cord-derived mesenchymal stem cells (UCMSCs) were inoculated onto a culture dish at a density of 3×10⁴/cm² in a DMEM-HG medium containing 10% FBS. 2 days later, the medium was replaced with a 1% FBS culture medium. Next day, the medium was replaced with a DMEM-HG medium containing 10% FBS and supplemented with 50 µg/mL ascorbic acid and 10 ng/mL connective tissue growth factor (CTGF) and differentiation was induced for 3, 7 or 14 days.

For fibrinogen-based 3D culture, Sylgard (Dow Corning, Midland, MI, USA) was coated on a 35-mm culture dish for 1-2 weeks and then two silk sutures of 5 mm in length were pinned on the Sylgard. After sterilizing the culture dish and sutures on a clean bench using 100% ethanol and UV and drying for 2 hours, they were immersed in DMEM for 1 hour at 37 °C in a CO₂ incubator. After mixing the gene-introduced MSCs and UCMSCs (2.25×10⁵ and 0.75×10⁵ cells respectively) at a ratio of 3:1 with a thrombin mixture (MEM supplemented with 10% fetal bovine serum, 1% antibiotic solution, 1 U/mL thrombin, 200 µM aminocaproic acid and 10 mg/mL aprotinin), a 10 mg/mL fibrinogen solution was added. After spreading the mixture on the Sylgard-coated culture dish quickly, it was left at 37 °C for 3 hours. Then, the cells were cultured for 3 days in a tendon cell differentiation medium (DMEM-HG containing 250 µM ascorbic acid and 50 µM L-proline). In order to investigate differentiation into tendon cells, the cells were stained with hematoxylin and eosin (H&E) and picrosirius red and subjected to immunohistochemical staining. In addition, the degree of differentiation was investigated from the expression of tendon cell marker genes. After fixing the cells in a 4% paraformaldehyde solution for 24 hours, 6-µm section samples were prepared by embedding in paraffin. Then, they were stained with H&E and picrosirius red. They were immunohistochemically stained by reacting with anti-Col I (ab34710, Abcam) and anti-Col III (ab7778, Abcam) diluted to 1:200 for a day in a refrigerator, followed by reaction with HRP-conjugated secondary antibodies at room temperature for 30 minutes.

### Isolation of RNAs from Zkscan8-introduced umbilical cord-derived mesenchymal stem cells (MSC-Zk8)

In order to investigate the expression of various genes upon introduction of Zkscan8, total RNAs were extracted using a HiYield total RNA mini kit (Real Biotech Corporation, Taiwan). After measuring absorbance at 260 nm and 280 nm using a spectrophotometer (NanoDrop, DE, USA), the total RNAs were quantified. Then, cDNAs were synthesized from 1 µg of the total RNAs using a Superscript II reverse transcriptase (Invitrogen, CA. USA).

### Reverse transcription polymerase chain reaction (RT-PCR)

Maxime^{™} PCR PreMix (i-StarTaq, iNtRON, Sungnam, Korea) was used to conduct reverse transcription polymerase chain reaction (RT-PCR). 16 µL of RNase-free distilled water, 2µL of cDNAs and 1 µL of primers per each were added and the reaction was conducted under the following conditions. Pre-denaturation was performed at 95 °C for 30 minutes, denaturation at 95 °C for 30 seconds, annealing at 55 °C for 30 seconds, and extension at 72 °C for 1 minute. After repeating this procedure for a total of 32 times, followed by cooling at 72 °C for 5 minutes, the bands of the obtained PCR product were observed on 1% agarose gel.

**[Table 1]**

| Target genes | | Primer sequences (5' → 3') | Product length (bp) |
|---|---|---|---|
| Zkscan8 | SEQ ID NO 4 | CGGAGGAAAGTCGGAAACCA | 198 |
| Zkscan8 | SEQ ID NO 5 | CTTCCCGTGGACCAAGAGTC | 198 |
| P PARγ2 | SEQ ID NO 6 | TTGGTGACTTTATGGAGCCC | 311 |
| P PARγ2 | SEQ ID NO 7 | CATGTCTGTCTCCGTCTTCT | 311 |
| aP2 | SEQ ID NO 8 | AAGAAGTAGGAGTGGGCTTT | 285 |
| aP2 | SEQ ID NO 9 | CCACCACCAGTTTATCATCC | 285 |
| Osteopontin | SEQ ID NO 10 | GAGACCCTTCCAAGTAAGTC | 354 |
| Osteopontin | SEQ ID NO 11 | GATGTCCTCGTCTGTAGCAT | 354 |
| ALP | SEQ ID NO 12 | TGGAGCTTCAGAAGCTCAAC | 454 |
| ALP | SEQ ID NO 13 | ATCTCGTTGTCTGAGTACCA | 454 |
| Sox6 | SEQ ID NO 14 | AACATGTGGCCTCCCATCTG | 300 |
| Sox6 | SEQ ID NO 15 | TCAGTGTGTCCACCACATCG | 300 |
| Aggrecan | SEQ ID NO 16 | TCAGGAGGGCTGGAACAAGT | 350 |
| Aggrecan | SEQ ID NO 17 | GGAGGTGGTAATTGCAGGGA | 350 |
| Mkx | SEQ ID NO 18 | GGCCACGAACACTACCATGA | 175 |
| Mkx | SEQ ID NO 19 | AGCTGCGCTTTCACCCTTAT | 175 |
| Egr-1 | SEQ ID NO 20 | CCAGTGGAGTCCTGTGATCG | 206 |
| Egr-1 | SEQ ID NO 21 | TCGCTCCTGGCAAACTTTCT | 206 |
| Egr-2 | SEQ ID NO 22 | TCTTCCTCTCTGGCCTACCC | 400 |
| Egr-2 | SEQ ID NO 23 | GTCTGTTGGGGTACTTGCGA | 400 |
| COL1a1 | SEQ ID NO 24 | AGTGGTTTGGATGGTGCCAA | 170 |
| COL1a1 | SEQ ID NO 25 | GCACCATCATTTCCACGAGC | 170 |
| COL3a1 | SEQ ID NO 26 | CTTCTCTCCAGCCGAGCTT | 191 |
| COL3a1 | SEQ ID NO 27 | CCAGTGTGTTTCGTGCAACC | 191 |
| Desmin | SEQ ID NO 28 | GAGGAAATCCGGCACCTCAA | 253 |
| Desmin | SEQ ID NO 29 | CATCCCCGTGTCTCGATGGTC | 253 |
| NEFL | SEQ ID NO 30 | CTGGAAATCGAAGCATGCCG | 363 |
| NEFL | SEQ ID NO 31 | GCGGGTGGACATCAGATAGG | 363 |
| GAPDH | SEQ ID NO 32 | AAATCCCATCACCATCTTCCAG | 313 |
| GAPDH | SEQ ID NO 33 | CATGAGTCCTTCCACGATACC | 313 |

### Quantitative reverse transcription polymerase chain reaction (quantitative RT-PCR)

The expression of the following genes was quantified in real time by quantitative reverse transcription polymerase chain reaction (quantitative RT-PCR; qRT-PCR) using Go Taq^{™} probe qPCR and RT-qPCR systems (Promega, WI, USA), TaqMan^{™} Gene Expression Assays (Applied Biosystems, Foster City, CA, USA) and LightCycler 480 (Roche Applied Science, Mannhein, Germany); Scx (Hs03054634_g1), Mkx (Hs00543190_m1), Egr-1 (Hs00152928_m1), Egr-2 (Hs00166165_m1), Thbs4 (Hs00170261_m1), GAPDH (Hs99999905_m1). The polymerase chain reaction was conducted as follows. After repeating 50 cycles of pre-denaturation at 95 °C for 10 minutes, denaturation at 95 °C for 15 seconds, annealing at 60 °C for 1 minute and extension at 72 °C for 4 seconds, the reaction mixture was cooled at 40 °C for 30 seconds. Melting curve analysis was performed using the 2-ΔCt calculation method, and the result of qRT-PCR was analyzed with reference to the expression of GAPDH [Livak KJ, Schmittgen TD. Analysis of relative gene expression data using real-time quantitative PCR and the 2-ΔΔCt method. Methods. 2001, 25:402-408].

### Isolation of proteins and western blotting

A tendon cell lysate was obtained from tendon cells as follows. After adding a lysis buffer (PRO-PREPTM, iNtRON, Sungnam, Korea) to tendon cell pellets, they were disrupted by vigorously vortexing for 10 seconds for 10 or more times. The resulting solution was centrifuged at 4 °C and at 13,000 rpm for 20 minutes, and a tendon cell lysate was obtained by recovering the supernatant. The tendon cell lysate was subjected to BCA for quantification of protein contents. The tendon cell lysates (10 µg) of the same concentration were loaded onto 10% acrylamide gel and proteins were separated based on size by electrophoresis (SDS-PAGE). The separated proteins were sufficiently soaked with a 1x transfer buffer (20% methanol, 0.025 M Tris base and 0.19 M glycine) and then transferred to a PVDF membrane by flowing current at 100 V for 90 minutes. The PVDF membrane was blocked for 1 hour in a 1x TBS-T solution (10 mM Tris pH7.5, 100 mM NaCl, 0.1% Tween 20) supplemented with 5% skim milk. Then, after adding primary antibodies to the 1x TBS-T solution at a ratio of 1:1000, reaction was performed by stirring at 4 °C. The used primary antibodies are as follows: Zkscan8 antibody (STJ26239, St John's Laboratory) and β-actin antibody (ab170325, Abcam). The PVDF membrane reacted with the primary antibodies were washed sufficiently with 1x TBS-T for 10 minutes for 3 times or more. Then, after adding secondary antibodies (diluted to 1:20,000) to the 1x TBS-T solution, reaction was performed for 1 hour by stirring at room temperature. The used secondary antibodies are as follows: goat anti-mouse IgG-HRP (SA001-500, GenDEPOT) and goat anti-rabbit IgG-HRP (7074S, Cell Signaling). After washing 3 times with a 1x TBS-T solution for 10 minutes and treating with ECL, the PVDF membrane was imaged with ImageQuant LAS4000 mini (GE Healthcare Life Sciences, Little Chalfont, UK).

### Design of animal experiment

All animal care and experimental procedures were approved by and conducted in accordance with the institutional experimental animal research committee (IACUC_2019_0044). 88 male Sprague-Dawley rats (12-week-old, 340-360 g) were divided into four groups (1) normal group (Normal); 2) physiological saline group (Saline); 3) umbilical cord-derived mesenchymal stem cell group (MSC); 4) Zkscan8-introduced umbilical cord-derived mesenchymal stem cell group (MSC-Zk8)).

The rats were anesthetized using Zoletil (30 mg/kg) and Rompun (10 mg/kg), and only the left shoulders of the rats were used in all the experiments. Surgery was conducted after slightly pressing the rat's sole with a fingernail to check if the anesthesia was successful. Then, the skin in front of the acromion of the left shoulder was incised by 2 cm. After exposing the supraspinatus tendon by incising the trapezius muscle and deltoid muscle attached to the acromion, a round full-thickness rupture tendon injury was made at a distance of 1 mm from the cartilage region of the supraspinatus tendon and the humeral head using a biopsy punch (BP-20F, Kai Medical Europe GmbH, Bremen, Germany) with a diameter of 2 mm (about 50% or larger of the tendon width). Then, 2) 10 µL of physiological saline, 3) umbilical cord-derived mesenchymal stem cells (1×10⁶ cells/10 µL physiological saline) or 4) Zkscan8-introduced umbilical cord-derived mesenchymal stem cells (1×10⁶ cells/10 µL physiological saline) was injected to both sides of the ruptured tendon in two divided dosages using a 30-G needle. After suturing the trapezius and deltoid muscles with a 4-0 Vicryl suture (W9074, Ethicon, Cincinnati, OH, USA) and suturing the skin with Black Silk (SK439, AILee, Busan, Korea), the wound site was disinfected. After the surgery, the rats were allowed free cage activity.

The rats were sacrificed at weeks 2 and 4 after the surgery. The supraspinatus tendon of the rats was harvested and used for macroscopic, histological and biomechanical evaluation.

### Macroscopic evaluation

At 2 and 4 weeks after the surgery, the rats were sacrificed in a carbon dioxide chamber. The supraspinatus tendon of the rats was harvested along with the humeral head without removing the supraspinatus muscle. For macroscopic evaluation of tendon regeneration, Stoll's modified semi-quantitative evaluation system was used [Stoll C, John T, Conrad C et al. Healing parameters in a rabbit partial tendon defect following tenocyte/biomaterial implantation. Biomaterials 2011; 32(21): 4806-4815]. The 12 parameters in the system were tendon rupture (breakage at the defective site), inflammation (swelling/redness or inflammation), tendon surface (tendon surface is not smooth but rough and uneven), neighboring tendon (the color, thickness and outer surface of the of tendon near the defective site turn abnormal), level of the defect (the defective site is filled and bulges as compared to the neighboring tendon), defect size (defect size is increased to 3 mm or larger), swelling/redness of tendon (the injured tendon turns red or swells), connection surrounding tissue and slidability (the injured tendon congeals with neighboring tissue without sliding (being separated) smoothly), tendon thickness (tendon thickness is increased as compared to the original thickness), color of tendon (bright white tendon has turn opaque and dark red), single strain of muscle (the supraspinatus tendon is intermixed with neighboring muscle and tissue, rather than only with the supraspinatus muscle), and transition of the construct to the surrounding healthy tissue (the connection between the defective site and neighboring healthy tendon is not smooth; the beginning of defect is clearly distinguished). Each parameter was given 0 or 1 point except for tendon swelling/redness (0 to 2) and tendon thickness (0 to 3). Therefore, the total macroscopic score varied between 0 (normal tendon) and 15 (most severe injury).

### Histological evaluation

After the macroscopic evaluation, the harvested tissues were immediately fixed in 4% (w/v) paraformaldehyde (PFA; Merck, Germany) for 24 hours, followed by decalcification in 10% EDTA (ethylendiaminetetraacetic acid, Sigma-Aldrich, St Louis, MO, USA) for 2 days. After the decalcification, the tissues were dehydrated through an increasing series of ethanol gradient and defatted in chloroform. The fixed tissues were embedded in paraffin blocks and carefully trimmed to the appropriate middle site of tendon using a microtome, cut into 4-cm-thick sections and attached onto slides.

A randomly selected slide was stained with hematoxylin and eosin (H&E) and analyzed with an optical microscope (U-TVO 63XC; Olympus Corp., Japan). For the evaluation of tendinopathy, each slide was evaluated using Astrom and Movin's modified semi-quantitative grading scale [Jo CH, Shin WH, Park JW et al. Degree of tendon degeneration and stage of rotator cuff disease. Knee Surg Sport Tr A 2017; 25 (7): 2100-2108]. The 7 parameters of the system include: fiber structure (long collagen fibers are broken into small pieces), fiber arrangement (collagen fibers that have been arranged in parallel are arranged irregularly), rounding of the nuclei (the nuclei of fibroblasts that have been flat due to inactivation turn round as they are injured or activated), variations in cellularity (the number of cells in the tendon is increased and the cells form several populations), increased vascularity (the number and size of blood vessels in the tendon are increased significantly), decreased stainability (fibers constituting the tendon are decreased and stainability is decreased due to the decreased fiber density) and hyalinization (the tendon tissues consisting of collagen fibers turn glassy). The total degeneration score varied between 0 (normal tendon) and 21 (most severely degenerated).

For analysis of collagen fiber coherence (the state where the collagen fibers constituting tendon are coherently aligned in parallel), the slides were stained with picrosirius red and images were obtained using a circular polarization optical microscope. The collage fiber coherence, which is a measure of the extent of collagen fiber alignment in the major axis of the tendon, was quantified using a computer program called Orientation J plug-in for imaged. Five regions were analyzed for each slide and the mean value multiplied by 100 was used as the final coherence value [Degen RM, Carbone A, Carballo C et al. The Effect of Purified Human Bone Marrow-Derived Mesenchymal Stem Cells on Rotator Cuff Tendon Healing in an Athymic Rat. Arthroscopy 2016; 32 (12): 2435-2443].

For evaluation of glycoaminoglycan (GAG)-rich area (formation of nonspecific cartilage tissue in tendon tissue) associated with heterotopic cartilage formation, the slides were stained with safranin O/fast green (Saf-O) and images were obtained using an optical microscope. The obtained images were analyzed with imageJ, and the glycoaminoglycan-rich area stained red was quantified.

The occurrence of heterotopic ossification (formation of nonspecific bone tissue in tendon tissue) was evaluated. It was evaluated that heterotopic ossification occurred when separated, clustered and bar-shaped foci were observed in the whole tendon structure on the slides stained with H&E. The area of heterotopic ossification was analyzed using imaged.

### Biomechanical evaluation

At 2 and 4 weeks after the surgery, the rats were sacrificed in a carbon dioxide chamber. The tendon of the rats was harvested with the supraspinatus muscle attached to the humerus. Then, only the tendon was left by carefully removing the muscle. The harvested tissues were wrapped in saline-soaked gauze and kept at - 80 °C. Before testing, the tissues were thawed with saline-wetted gauze for 24 hours. The tissues were kept moist using saline-wetted gauze during all tests. The distal part of the humerus was vertically embedded in an aluminum tube full of polymethylmethacrylate (PMMA) in the custom-designed lower jig of a testing system. The proximal end of the tendon was fixed to the upper jig and compressed with sandpaper, gauge and rubber to prevent slippage and minimize damage of specimens. Testing was performed at 90° of abduction using a material testing system (H5K5, Tinus Olsen, England, UK) (FIG. 13). All specimens were loaded at a constant rate of 0.1 mm/s. The slippage of the tendon was inspected visually. The cross-sectional area of the tendon was measured at the center of the defect region and was calculated with the formula 'area = abπ/4'. The ultimate failure load (maximum load that the tendon can bear until it is broken), stiffness (maximum force that the tendon can endure without deformation) and ultimate stress (ultimate failure load per unit area; maximum load that the tendon can bear until it is broken per unit area) were calculated from the load-displacement curve [Galatz L, Silva M, Rothermich S et al. Nicotine delays tendon-to-bone healing in a rat shoulder model. JBJS 2006; 88 (9): 2027-2034.].

### Statistical analysis

All data were shown as mean ± SD. The data were analyzed by one-way analysis of variance (ANOVA) and post-hoc analysis was performed using Bonferroni multiple comparison test. T-test was used to compare two groups. All statistical analyses were performed using the SPSS software version 23 (IBM). Differences of p < 0.05 were considered statistically significant.

### Experimental results

### Whole transcriptome profiling

For analysis of the expression patterns of tenogenesis, myogenesis, chondrogenesis and osteogenesis markers during embryonic stages, the expression patterns of a total of 25,070 expressed genes in embryonic stages E9.5-E15.5 were analyzed from whole transcriptome expression profiling datasets GSE30138, GSE54207 and GSE65180. FIG. 2 summarizes the distribution of the datasets during embryogenesis (stages E9.5-E15.5).

As seen from FIG. 2, a total of 9,163 DEGs (differentially expressed genes) were identified. Among them, 149 genes showing similar expression patterns as 28 markers were identified as being involved in tenogenesis, myogenesis, osteogenesis and chondrogenesis.

### Analysis of gene expression pattern and PPI network during embryogenesis

Because analysis was conducted using three datasets, the similarity of expression patterns to marker genes was compared based on distance scores of the genes. 0 point was given if the expression pattern was identical to that of the marker genes in the three datasets. 0.33 point and 0.67 were given, respectively, if it is different in one and two datasets when the expression pattern began to increase. And, -0.33 point and -0.67 were given, respectively, if it is different in one and two datasets when the expression pattern began to decrease. Among the 9,163 DEGs, 149 genes with total distance scores of ±0.33 or lower, i.e., those showing difference in expression patterns in only one embryogenic stage, were screened primarily. The primarily screened candidate genes were subjected to PPI network, functional enrichment and module analyses while adjusting degree cut-off.

As a result of the protein-protein interaction (PPI) network analysis, three sub-clusters were identified. 99 genes were screened secondarily by excluding well-known genes. Then, 42 genes functionally related to tendon regeneration were screened tertiarily through gene ontology (GO). The tertiarily screened 42 genes were used for the final screening of genes related with tendon regeneration.

### Comparison of expression pattern of tenogenesis markers and Zkscan8 depending on embryogenic stages

FIG. 3a shows a result of analyzing the expression pattern of tenogenesis markers and Zkscan8 depending on the stages of embryogenesis.

The expression pattern of the Zkscan8 gene was compared with that of tenogenesis markers depending on embryogenic stages to analyze their similarity. As shown in FIG. 3a, Sex and Tgif1 were increased continuously from E9.5 and were decreased at E13.5. Mkx was increased continuously until E13.5, and Tgf-β2 showed consistently high expression levels from E9.5 to until E13.5. Egr1 and Thbs4 were decreased at E10.5-E11.5 as compared to at E9.5 and then were increased again from E12.5. Epha4 was increased remarkably from E10.5. The expression of Zkscan8 was increased at E10.5 and the expression pattern was generally similar to that of the tendon markers.

### Analysis of Zkscan8 expression pattern in normal tendon and tendon with rotator cuff disease

The expression patterns of tenogenesis markers and the Zkscan8 gene in the normal tendon obtained from a normal human and the tendon obtained from a patient with rotator cuff disease were investigated.

FIG. 3b shows a result of measuring the expression pattern of the tenogenesis markers and Zkscan8 in the normal human tendon (Normal) and the tendon of a patient with rotator cuff disease (Tendinopathy).

As shown in FIG. 3b, the expression of Tnmd and Thbs4, known as tenogenesis markers, was increased significantly in the tendon with rotator cuff disease by about 5.4 times and 2 times, respectively. The expression of Col3a1 was increased significantly in the tendon with rotator cuff disease by 3.2 times as compared to the normal tendon. In addition, the expression of MMP-9 was also increased by 11.8 times in the tendon with rotator cuff disease as compared to the normal tendon. However, the expression of the Zkscan8 gene was specifically in the tendon with rotator cuff disease as compared to the normal tendon. Through this, it was confirmed that Zkscan8 can be used as a gene for preventing or treating rotator cuff disease. Zkscan8 was selected finally.

### Characterization of Zkscan8-introduced umbilical cord-derived mesenchymal stem cells (MSC-Zk8)

A pscAAV-GFP vector, which is a control group vector, and a pscAAV vector wherein Zkscan8 is inserted, are schematically shown in FIG. 1c. Umbilical cord-derived mesenchymal stem cells into which pscAAV-GFP and pscAAV-Zkscan8 were introduced were prepared as described above, and the characteristics of the Zkscan8-introduced umbilical cord-derived mesenchymal stem cells (MSC-Zk8) were compared with a control group (MSC-GFP). For this, cell morphology and proliferation ability were compared.

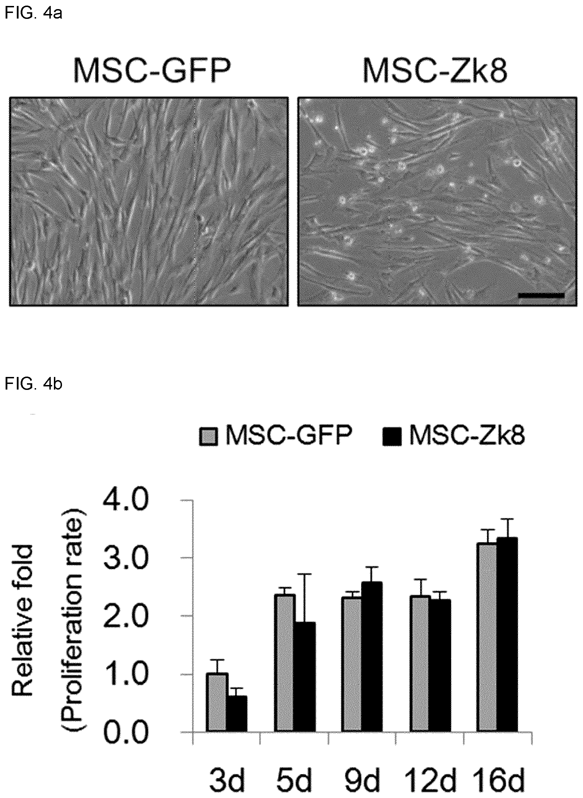

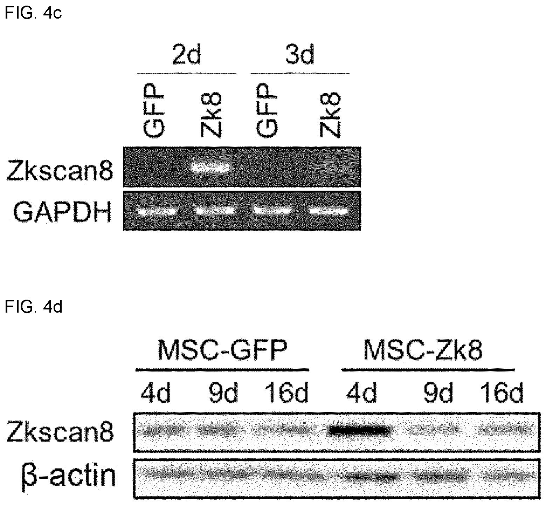

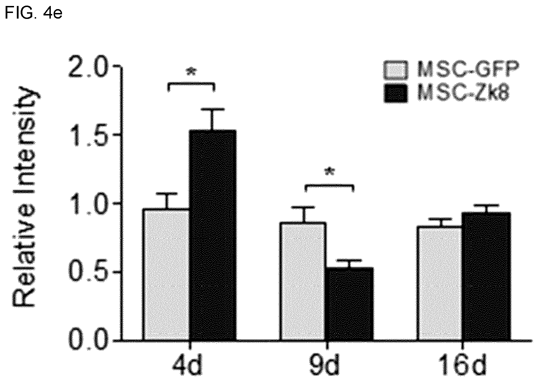

FIG. 4 shows the result of analyzing the characteristics (cell morphology and proliferation ability) of the Zkscan8-introduced umbilical cord-derived mesenchymal stem cells (MSC-Zk8) and the control group (MSC-GFP). FIG. 4a shows the microscopic images of the Zkscan8-introduced umbilical cord-derived mesenchymal stem cells (MSC-Zk8) and the control group (MSC-GFP), FIG. 4b shows a result of quantifying the proliferation ability of the Zkscan8-introduced umbilical cord-derived mesenchymal stem cells (MSC-Zk8) and the control group (MSC-GFP), FIG. 4c shows a result of measuring the expression of the Zkscan8 gene in the Zkscan8-introduced umbilical cord-derived mesenchymal stem cells (MSC-Zk8) and the control group (MSC-GFP) by RT-PCR, and FIG. 4d and FIG. 4e show a result of analyzing the expression of the Zkscan8 protein in the Zkscan8-introduced umbilical cord-derived mesenchymal stem cells (MSC-Zk8) and the control group (MSC-GFP) by western blotting.

As shown in FIGS. 4a and 4b, the Zkscan8-introduced umbilical cord-derived mesenchymal stem cells (MSC-Zk8) and the control group (MSC-GFP) showed the characteristic morphology of fibroblasts. That is to say, the two cells were found to have no difference in proliferation ability.

As shown in FIGS. 4c-4e, the expression level of Zkscan8 over time was analyzed by extracting total RNAs and proteins from the Zkscan8-introduced umbilical cord-derived mesenchymal stem cells (MSC-Zk8) and the control group (MSC-GFP).

From the RT-PCR result, it can be seen that Zkscan8 was overexpressed in the Zkscan8-introduced umbilical cord-derived mesenchymal stem cells (MSC-Zk8) and the control group (MSC-GFP) from 2 days after the introduction of the gene and the expression was decreased after 3 days.

From the western blotting result, it was confirmed that the expression of the Zkscan8 protein was increased in the Zkscan8-introduced umbilical cord-derived mesenchymal stem cells (MSC-Zk8) by 1.5 times or higher as compared to the control group (MSC-GFP). Accordingly, it was confirmed that human umbilical cord-derived mesenchymal stem cells overexpressing the Zkscan8 protein were prepared successfully.

### Evaluation of differentiability of Zkscan8-introduced umbilical cord-derived mesenchymal stem cells (MSC-Zk8) to tendon

In order to investigate the relationship between the Zkscan8 gene and tendon differentiation, the umbilical cord-derived mesenchymal stem cells (UCMSCs) were cultured in a tendon differentiation medium containing connective tissue growth factor (CTGF) and ascorbic acid for 2 weeks to induce differentiation into tendon cells. The expression of Zkscan8 in the cells was investigated on days 3, 7 and 14. Then, after culturing the Zkscan8-introduced umbilical cord-derived mesenchymal stem cells (MSC-Zk8) and the control group (MSC-GFP) for 3 days by 3D culture to induce differentiation into tendon, the expression of tendon-related genes (Scx, Egr-1, Egr-2, Thbs4) was confirmed by qRT-PCR and histological analysis.

FIGS. 5a-5d show a result of evaluating the effect of Zkscan8 overexpression on tendon differentiation. FIG. 5a shows a result of culturing the umbilical cord-derived mesenchymal stem cells (UCMSCs) in a tendon differentiation medium for 2 weeks and then quantifying the expression level of Zkscan8 over time by RT-PCR.

As shown in FIG. 5a, the expression of Zkscan8 was significantly increased in the umbilical cord-derived mesenchymal stem cells (UCMSCs) on day 14 after the induction of tendon differentiation by 1.5 times or higher. This shows that there is a relationship between tendon differentiation and Zkscan8 expression.

At present, several transcription factors such as Sex, Mkx and Egr-1/2 are known to be involved in tendon development. In upper limbs, Sex and Sox9 are the first signals for tendon progenitor cell initiation, whereas Mkx and Egr-1/2 are the second signals for tendon differentiation and maturation. In particular, Egr-1/2 is known as an essential transcription factor for tendon differentiation. It has been found as a new DNA-binding protein involved in tendon differentiation in vertebrates by regulating the production of type 1 collagen. The expression pattern of tenogenesis-related transcription factors (scleraxis; Sex, mohawk; Mkx, early growth response-1/2; Egr-1/2) in the Zkscan8-introduced umbilical cord-derived mesenchymal stem cells (MSC-Zk8) and the control group (MSC-GFP) over time was investigated by qRT-PCR.

FIG. 5b shows a result of culturing the Zkscan8-introduced umbilical cord-derived mesenchymal stem cells (MSC-Zk8) and the control group (MSC-GFP) for 7 days and then analyzing the expression level of the tenogenic transcription factors (Scx, Mkx, Egr-1, Egr-2) over time by qRT-PCR. The relative expression levels with respect to the control group (MSC-GFP) on day 2 are shown in the graphs.

As shown in FIG. 5b, the expression of Sex in the Zkscan8-introduced umbilical cord-derived mesenchymal stem cells (MSC-Zk8) was increased by 1.61 times from day 3, and the expression of Egr-1 and Egr-2 was increased by 2.93 times and 4 times, respectively, on day 2. In contrast, no change was found for the control group (MSC-GFP).

The change in histological pattern and tendon differentiation markers of the Zkscan8-introduced umbilical cord-derived mesenchymal stem cells (MSC-Zk8) when the cells were 3D-cultured under uniaxial tension to induce differentiation into tendon and ligament was investigated.

FIG. 5c and FIG. 5d show a result of culturing the Zkscan8-introduced umbilical cord-derived mesenchymal stem cells (MSC-Zk8) and the control group (MSC-GFP) in a tendon differentiation medium for 3 days through 3D culture and then quantifying the tendon-related markers (Scx, Egr-1, Egr-2, Thbs4) by qRT-PCR.

As shown in FIG. 5c and FIG. 5d, the expression of all of Sex (1.7 times), Egr-1 (2.7 times), Egr-2 (2.8 times) and Thbs4 (1.5 times) was increased in the Zkscan8-introduced umbilical cord-derived mesenchymal stem cells (MSC-Zk8) that had been 3D-cultured for 3 days.

FIG. 5e show a result of 3D-culturing the Zkscan8-introduced umbilical cord-derived mesenchymal stem cells (MSC-Zk8) and the control group (MSC-GFP) in a tendon differentiation medium for 3 days to induce tendon differentiation and then analyzing histological characteristics.

As shown in FIG. 5e, the Zkscan8-introduced umbilical cord-derived mesenchymal stem cells (MSC-Zk8) showed well-aligned thin and long cells. In addition, the presence of type 1 collagen in the matrix of the Zkscan8-introduced umbilical cord-derived mesenchymal stem cells (MSC-Zk8) was confirmed because they were stained yellow by picrosirius red staining. In addition, the expression of type 1 collagen and type 3 collagen was confirmed through immunohistochemical staining using type 1 collagen and type 3 collagen antibodies. In particular, it was confirmed that the expression of type 1 collagen was significantly higher in the Zkscan8-introduced umbilical cord-derived mesenchymal stem cells (MSC-Zk8). In conclusion, it can be seen that Zkscan8 is directly involved in the differentiation into tendon cells.

### Analysis of expression pattern of fat, bone, cartilage, tendon, muscle and nerve-related markers in Zkscan8-introduced umbilical cord-derived mesenchymal stem cells (MSC-Zk8)

Tendon tissue has many similarities to bone, cartilage and muscle tissues. Therefore, it was investigated whether Zkscan8 also affects the markers of fat, bone, cartilage, tendon, muscle and nerve that constitute the musculoskeletal system, in addition to tendon tissue. Specifically, Zkscan8-introduced umbilical cord-derived mesenchymal stem cells (MSC-Zk8) were prepared and the expression pattern of various genes including musculoskeletal markers was analyzed 2 and 3 days later.

FIG. 6 shows a result of analyzing the expression pattern of fat, bone, cartilage, tendon, muscle and nerve cell-related markers in the Zkscan8-introduced umbilical cord-derived mesenchymal stem cells (MSC-Zk8) and the control group (MSC-GFP). The expression of the fat, bone, cartilage, tendon, muscle and nerve-related genes was increased on the whole in the Zkscan8-introduced umbilical cord-derived mesenchymal stem cells (MSC-Zk8). In particular, the expression of ALP (alkaline phosphatase), Sox6 (SRY-box transcription factor 6), Egr-2, Col1A1, desmin and NEFL (neurofilament light polypeptide) in the Zkscan8-introduced umbilical cord-derived mesenchymal stem cells (MSC-Zk8) was remarkably increased on day 3 as compared to the control group (MSC-GFP). This suggests that the overexpression of Zkscan8 affects the musculoskeletal system including bone, cartilage, muscle and nerve.

### Macroscopic evaluation

FIG. 7a shows the macroscopic images of the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. The tissues around the defective region were removed for easier observation of the defect state of the tendon.

FIG. 7b shows a result of analyzing total macroscopic score for the supraspinatus tendon of the normal group (Normal), the physiological saline group (Saline), the umbilical cord-derived mesenchymal stem cell group (MSC) and the Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).

As shown in FIG. 7a and FIG. 7b, the total macroscopic score, which is a measure of apparently severe injury, was compared between the groups. At week 2, the total macroscopic score was 4.75 ± 0.46 for the Zkscan8-introduced umbilical cord-derived mesenchymal stem cell group (MSC-Zk8), but 10.75 ± 1.28 (p < 0.000) and 7.25 ± 0.89 (p < 0.000) for the physiological saline group and the umbilical cord-derived mesenchymal stem cell group, respectively, indicating severe injury. In particular, the Zkscan8-introduced umbilical cord-derived mesenchymal stem cell group (MSC-Zk8) showed lower scores (less injury) in inflammation, connection surrounding tissue and slidability and tendon thickness as compared to other groups.

At week 4, the total macroscopic score of the Zkscan8-introduced umbilical cord-derived mesenchymal stem cell group (MSC-Zk8) was 2.75 ± 0.46. In contrast, the total macroscopic score was 9.00 ± 0.00 (p < 0.000) and 4.25 ± 0.89 (p < 0.000), respectively, for the physiological saline group and the umbilical cord-derived mesenchymal stem cell group, indicating remarkably sever injury as compared to MSC-Zk8. The MSC-Zk8 group showed less tendon swelling and redness as compared to other groups.

From these experiments, it can be seen that the overexpression of Zkscan8 significantly increases the ability of recovering from tissue injury the umbilical cord-derived mesenchymal stem cells.

When the rotator cuff is injured, adhesion occurs between the rotator cuff and surrounding tissues. This limits the motion of the patient and causes severe pain by aggravating the tendon injury. It can be seen that the pain and symptoms in a patient that may be caused by tendon injury can be prevented or treated in the Zkscan8-introduced umbilical cord-derived mesenchymal stem cell group (MSC-Zk8).

### Histological evaluation

FIG. 8a shows the H&E staining images of the supraspinatus tendon of the normal group (Normal), the physiological saline group (Saline), the umbilical cord-derived mesenchymal stem cell group (MSC) and the Zkscan8-introduced umbilical cord-derived mesenchymal stem cell group (MSC-Zk8) at weeks 2 and 4 after the beginning of experiment (magnification: x100), and FIG. 8b shows a result of analyzing total degeneration score for the supraspinatus tendon of the normal group (Normal), the physiological saline group (Saline), the umbilical cord-derived mesenchymal stem cell group (MSC) and the Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).

As shown in FIG. 8a and FIG. 8b, the total degeneration score of the Zkscan8-introduced umbilical cord-derived mesenchymal stem cell group (MSC-Zk8) was 13.00 ± 1.20 at week 2 after the beginning of experiment. In contrast, the total degeneration score was 17.75 ± 0.46 (p < 0.001) and 16.00 ± 0.93 (p < 0.001) for the physiological saline group (Saline) and the umbilical cord-derived mesenchymal stem cell group (MSC), respectively. That is to say, the Zkscan8-introduced umbilical cord-derived mesenchymal stem cell group (MSC-Zk8) showed significantly less degenerative changes of tendon tissue as compared to other groups. In particular, the Zkscan8-introduced umbilical cord-derived mesenchymal stem cell group (MSC-Zk8) showed significant recovery in fiber arrangement and increased vascularity as compared to the umbilical cord-derived mesenchymal stem cell group (MSC).

At week 4, the total degeneration score was 7.00 ± 1.07 for the Zkscan8-introduced umbilical cord-derived mesenchymal stem cell group (MSC-Zk8), and 16.25 ± 0.89 (p < 0.01) and 8.75 ± 1.83 (p = 0.05) for the physiological saline group (Saline) and the umbilical cord-derived mesenchymal stem cell group (MSC), respectively. The Zkscan8-introduced umbilical cord-derived mesenchymal stem cell group (MSC-Zk8) showed significantly lower degeneration of tendon as compared to other groups.

FIG. 9a shows the PSR staining images of the supraspinatus tendon of the normal group (Normal), the physiological saline group (Saline), the umbilical cord-derived mesenchymal stem cell group (MSC) and the Zkscan8-introduced umbilical cord-derived mesenchymal stem cell group (MSC-Zk8) at weeks 2 and 4 after the beginning of experiment (magnification: x100), and FIG. 9b shows a result of analyzing collage fiber coherence for the supraspinatus tendon of the normal group (Normal), the physiological saline group (Saline), an umbilical cord-derived mesenchymal stem cell group (MSC) and the Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).

As shown in FIG. 9a and FIG. 9b, at week 2 after the beginning of experiment, the collage fiber coherence score of the Zkscan8-introduced umbilical cord-derived mesenchymal stem cell group (MSC-Zk8) was 43.35 ± 8.35. In contrast, the collage fiber coherence score was 20.69 ± 4.88 (p = 0.003) and 27.61 ± 6.97 (p = 0.042) for the physiological saline group (Saline) and the umbilical cord-derived mesenchymal stem cell group (MSC), respectively. It can be seen that the collage fiber coherence was significantly improved in the Zkscan8-introduced umbilical cord-derived mesenchymal stem cell group (MSC-Zk8) as compared to other groups.

At week 4 after the beginning of experiment, the collage fiber coherence score of the Zkscan8-introduced umbilical cord-derived mesenchymal stem cell group (MSC-Zk8) was significantly than other groups as 47.76 ± 5.77. Specifically, the collage fiber coherence score of the physiological saline group (Saline) was 19.86 ± 2.97 (p < 0.000).

FIG. 10a shows the Saf-O staining images of the supraspinatus tendon of the normal group (Normal), the physiological saline group (Saline), the umbilical cord-derived mesenchymal stem cell group (MSC) and the Zkscan8-introduced umbilical cord-derived mesenchymal stem cell group (MSC-Zk8) at weeks 2 and 4 after the beginning of experiment (magnification: x100), and FIG. 10b shows a result of analyzing glycoaminoglycan (GAG)-rich area for the supraspinatus tendon of the normal group (Normal), the physiological saline group (Saline), the umbilical cord-derived mesenchymal stem cell group (MSC) and the Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).

As shown in FIG. 10a and FIG. 10b, at week 2 after the beginning of experiment, the difference in the glycoaminoglycan-rich area was small among the groups. But, at week 4 after the beginning of experiment, the GAG-rich area of the Zkscan8-introduced umbilical cord-derived mesenchymal stem cell group (MSC-Zk8) was 39.17 ± 28.34 mm² and was 643.39 ± 156.99 mm² (p < 0.000) and 255.33 ± 107.01 mm² (p = 0.013) for the physiological saline group (Saline) and the umbilical cord-derived mesenchymal stem cell group (MSC), respectively. That is to say, the extent of heterotopic cartilage formation was significantly lower in the Zkscan8-introduced umbilical cord-derived mesenchymal stem cell group (MSC-Zk8) as compared to other groups. Especially, the score of the Zkscan8-introduced umbilical cord-derived mesenchymal stem cell group (MSC-Zk8) was not significantly different from that of the normal group.

FIG. 11 shows a result of analyzing area of ossification for the supraspinatus tendon of the normal group (Normal), the physiological saline group (Saline), the umbilical cord-derived mesenchymal stem cell group (MSC) and the Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).

As shown in FIG. 11, nonspecific bone formation did not occur in all the groups including the Zkscan8-introduced umbilical cord-derived mesenchymal stem cell group (MSC-Zk8).

From the above experimental results, it can be seen that the Zkscan8-introduced umbilical cord-derived mesenchymal stem cells can be used for clinical application in order to prevent or treat musculoskeletal diseases by promoting the regeneration of tendon tissues and helping the recovery of tendon tissues.

The treatment of the musculoskeletal system including tendon is unsuccessful mostly because the injured tendon is not recovered to normal tissue but replaced by scar tissue having irregular collagen fibers and many blood vessels. However, the treatment with the Zkscan8-overexpressed umbilical cord-derived mesenchymal stem cells resulted in increased alignment of collagen fibers as compared to when only the umbilical cord-derived mesenchymal stem cells were treated, so that the tendon at the defective site could be recovered to normal tendon tissue rather than being replaced by scar tissue. In addition, the clinical application of stem cells for treatment of the musculoskeletal system has been limited due to the increased risk of shoulder pain, re-rupture and complication as heterotopic cartilage formation and ossification are induced. However, the Zkscan8-overexpressed umbilical cord-derived mesenchymal stem cells suppressed heterotopic cartilage formation to a level comparable to that of the normal group. That is to say, Zkscan8 enables umbilical cord-derived mesenchymal stem cells to be clinically applicable by resolving the side effect. In addition, since it has the effect of preventing, alleviating and treating tendon tissue injury, it can be used for prevention and treatment of musculoskeletal diseases.

### Biomechanical evaluation

FIG. 12 and FIGS. 13a-13d describe biomechanical test for the regenerated tendon of the normal group (Normal), the physiological saline group (Saline), the umbilical cord-derived mesenchymal stem cell group (MSC) and the Zkscan8-introduced umbilical cord-derived mesenchymal stem cell group (MSC-Zk8) at weeks 2 and 4 after the beginning of experiment.

FIG. 12 shows sampling from the normal group (Normal), the physiological saline group (Saline), the umbilical cord-derived mesenchymal stem cell group (MSC) and the Zkscan8-introduced umbilical cord-derived mesenchymal stem cell group (MSC-Zk8) and the procedure of the biomechanical test.

FIG. 13a shows a result of analyzing ultimate failure load for the regenerated tendon taken from the normal group (Normal), the physiological saline group (Saline), the umbilical cord-derived mesenchymal stem cell group (MSC) and the Zkscan8-introduced umbilical cord-derived mesenchymal stem cell group (MSC-Zk8). All quantitative data are presented as mean ± standard error (S.E.M) (*p < 0.05).

As shown in FIG. 13a, the ultimate failure load of the Zkscan8-introduced umbilical cord-derived mesenchymal stem cell group (MSC-Zk8) was 17.54 ± 2.52 at 2 weeks after the surgery. The ultimate failure load was significantly higher for the MSC-Zk8 as compared to the physiological saline group (Saline) with 10.93 ± 1.62 (p < 0.001). The ultimate failure load of the umbilical cord-derived mesenchymal stem cell group (MSC) was 16.68 ± 3.01 and there was no significant difference from the MSC-Zk8 group.

At 4 weeks after the surgery, the ultimate failure load of the Zkscan8-introduced umbilical cord-derived mesenchymal stem cell group (MSC-Zk8) was 22.84 ± 1.99. The ultimate failure load was 18.51 ± 1.97 (p = 0.016) for the physiological saline group (Saline) and 22.14 ± 2.19 for the umbilical cord-derived mesenchymal stem cell group (MSC). That is to say, the tendon of the Zkscan8-introduced umbilical cord-derived mesenchymal stem cell group (MSC-Zk8) showed significant enhancement and recovery as compared to the physiological saline group (Saline).

FIG. 13b shows a result of analyzing stiffness for the regenerated tendon taken from the normal group (Normal), the physiological saline group (Saline), the umbilical cord-derived mesenchymal stem cell group (MSC) and the Zkscan8-introduced umbilical cord-derived mesenchymal stem cell group (MSC-Zk8). All quantitative data are presented as mean ± standard error (S.E.M) (*p < 0.05).

As shown in FIG. 13b, at 2 weeks after the surgery, the stiffness of the Zkscan8-introduced umbilical cord-derived mesenchymal stem cell group (MSC-Zk8) was 7.50 ± 0.60. The stiffness was 5.17 ± 0.55 (p = 0.001) and 6.83 ± 0.99 for the physiological saline group (Saline) and the umbilical cord-derived mesenchymal stem cell group (MSC), respectively. That is to say, it can be seen that the tendon of the Zkscan8-introduced umbilical cord-derived mesenchymal stem cell group (MSC-Zk8) was enhanced significantly as compared to the physiological saline group (Saline).

At 4 weeks after the surgery, the stiffness of the Zkscan8-introduced umbilical cord-derived mesenchymal stem cell group (MSC-Zk8) was 9.04 ± 1.12. The stiffness was 7.22 ± 0.62 (p = 0.013) and 7.38 ± 1.09 (p = 0.029) for the physiological saline group (Saline) and the umbilical cord-derived mesenchymal stem cell group (MSC), respectively. That is to say, it can be seen that the stiffness was significantly increased in the Zkscan8-introduced umbilical cord-derived mesenchymal stem cell group (MSC-Zk8) as compared to the physiological saline group (Saline) or the umbilical cord-derived mesenchymal stem cell group (MSC).

FIG. 13c shows a result of analyzing ultimate stress for the regenerated tendon taken from a normal group (Normal), the physiological saline group (Saline), the umbilical cord-derived mesenchymal stem cell group (MSC) and the Zkscan8-introduced umbilical cord-derived mesenchymal stem cell group (MSC-Zk8). All quantitative data are presented as mean ± standard error (S.E.M) (*p < 0.05).

FIG. 13d shows a result of analyzing cross-sectional area for the regenerated tendon taken from the normal group (Normal), the physiological saline group (Saline), the umbilical cord-derived mesenchymal stem cell group (MSC) and the Zkscan8-introduced umbilical cord-derived mesenchymal stem cell group (MSC-Zk8). All quantitative data are presented as mean ± standard error (S.E.M) (*p < 0.05).

As shown in FIG. 13c and FIG. 13d, there was no significant difference in the ultimate stress and cross-sectional area of the tendon at 2 and 4 weeks after the surgery.

Tendon is the tissue which transmits the force of muscle to bone during the movement of the body. In the present disclosure, the "ultimate failure load" refers to the maximum load that the tendon can bear until it is broken, and the "stiffness" refers to the maximum force that the tendon can endure without deformation.

It was confirmed that the tendon treated with the Zkscan8-overexpressed umbilical cord-derived mesenchymal stem cells showed increase in ultimate failure load and stiffness as compared to the tendon treated only with the umbilical cord-derived stem cells. This result is consistent with the preceding results that the treatment of the Zkscan8-overexpressed umbilical cord-derived mesenchymal stem cells to injured tendon exhibits the effect of inhibiting inflammation of tendon tissue, preventing degenerative changes, recovering collagen fiber arrangement and inhibiting heterotopic cartilage formation. That is to say, when the Zkscan8-overexpressed umbilical cord-derived mesenchymal stem cells are applied to fullthickness rupture injury, they can regenerate the defective tissue of the injured tendon and improve tendon functions.

## Claims

1. A composition for diagnosing a musculoskeletal disease, comprising a Zkscan8 (zinc finger protein with KRAB and SCAN domains 8) gene or an agent for measuring the expression level of a protein encoded by the gene as an active ingredient.

2. The composition according to claim 1, wherein the agent is a primer or a probe binding specifically to the Zkscan8 gene.

3. The composition according to claim 1, wherein the agent is an antibody or an aptamer binding specifically to a protein encoded by the Zkscan8 gene.

4. The composition according to claim 1, wherein the musculoskeletal disease is a tendon disease or a ligament disease.

5. A method for providing information necessary for diagnosing a musculoskeletal disease, comprising:
1) a step of measuring the expression level of a Zkscan8 (zinc finger protein with KRAB and SCAN domains 8) gene or a protein encoded by the gene in a biological sample isolated from a subject suspected of a musculoskeletal disease; and
2) a step of comparing the measured expression level of the Zkscan8 gene or the protein encoded by the gene with the expression level of a normal control group sample.

6. A composition for preventing or treating a musculoskeletal disease, comprising a Zkscan8 (zinc finger protein with KRAB and SCAN domains 8) protein as an active ingredient.

7. The composition according to claim 6, wherein the Zkscan8 protein has an amino acid sequence represented by SEQ ID NO 2.

8. The composition according to claim 6, wherein the musculoskeletal disease is a tendon disease or a ligament disease.

9. The composition according to claim 8, wherein
the tendon disease is one or more selected from a group consisting of tendon injury, tendon rupture, tendinitis, tendinosis and tendosynovitis, and
the ligament disease is one or more selected from a group consisting of knee quadriceps ligament rupture, coronary ligament sprain, collateral ligament rupture, anterior cruciate ligament injury, posterior cruciate ligament injury, ankle ligament injury, ligament rupture and sprain.

10. A pharmaceutical composition for preventing or treating a musculoskeletal disease, comprising a Zkscan8 (zinc finger protein with KRAB and SCAN domains 8) gene, a vector comprising the gene, a cell comprising the vector or a culture thereof as an active ingredient.

11. The composition according to claim 10, wherein the vector is a linear DNA, a plasmid DNA or a recombinant viral vector.

12. The composition according to claim 11, wherein the recombinant virus is any one selected from a group consisting of retrovirus, adenovirus, adeno-associated virus, herpes simplex virus and lentivirus.

13. The composition according to claim 10, wherein the cell is one or more selected from a group consisting of a stem cell, a dendritic cell, an autologous tumor cell and an established tumor cell.

14. The composition according to claim 13, wherein the stem cell is a mesenchymal stem cell.

15. The composition according to claim 14, wherein the mesenchymal stem cell is an umbilical cord-derived mesenchymal stem cell.

16. The composition according to claim 10, wherein the musculoskeletal disease is a tendon disease or a ligament disease.

17. The composition according to claim 16, wherein
the tendon disease is one or more selected from a group consisting of tendon injury, tendon rupture, tendinitis, tendinosis and tendosynovitis, and
the ligament disease is one or more selected from a group consisting of knee quadriceps ligament rupture, coronary ligament sprain, collateral ligament rupture, anterior cruciate ligament injury, posterior cruciate ligament injury, ankle ligament injury, ligament rupture and sprain.

18. The composition according to claim 10, wherein the composition increases the expression or activity of Scx (scleraxis), Mkx (mohawk), Egr-1 (early growth response-1) and Egr-2 (early growth response-2).

19. A method for screening a composition for preventing or treating a musculoskeletal disease, comprising:
(a) a step of contacting injured tendon, ligament, muscle, cartilage or bone cells with a test substance; and
(b) a step of measuring the expression level of a Zkscan8 (zinc finger protein with KRAB and SCAN domains 8) gene or protein in the sample,
wherein, if the expression level of the Zkscan8 is increased as compared to a normal control group in the step (b), the test substance is determined as a composition for preventing or treating a musculoskeletal disease.

20. The method according to claim 19, wherein the musculoskeletal disease is a tendon disease or a ligament disease.
